# EUROPÄISCHE PATENTSCHRIFT

(11) **EP 1 509 216 B1**
(45) Veröffentlichungstag und Bekanntmachung des Hinweises auf die Patenterteilung: **07.05.2008**
(21) Anmeldenummer: 03732544.6
(22) Anmeldetag: 06.06.2003
(51) Int. Cl.: A61K 31/13, A61K 31/60, A61K 31/192, A61K 31/136, A61K 31/12, A61K 31/405, C07C 59/64, C07C 229/42, C07C 69/86, C07D 209/28, C07C 51/487, C07D 417/12, C07D 231/34

(54) **WIRKSTOFFSALZE UND ESTER VON 1-DIMETHYLAMINO-3-(3-METHOXY-PHENYL)-2-METHYL-PENTAN-3-OL UND 3-(3-DIMETHYLAMINO-1-ETHYL-1-HYDROXY-2-METHYL-PROPYL)-PHENOL**
ACTIVE INGREDIENT SALTS AND ESTERS OF 1-DIMETHYLAMINO-3-(3-METHOXY-PHENYL)-2-METHYL-PENTAN-3-OL AND 3-(3-DIMETHYLAMINO-1-ETHYL-1-HYDROXY-2-METHYL-PROPYL)-PHENOL
SELS DE PRINCIPES ACTIFS ET ESTERS DE 1-DIMETHYLAMINO-3-(3-METHOXY-PHENYL)-2-METHYL-PENTAN-3-OL ET DE 3-(3-DIMETHYLAMINO-1-ETHYL-1-HYDROXY-2-METHYL-PROPYL)-PHENOL

(30) Priorität: 06.06.2002 DE 10225315
(43) Veröffentlichungstag der Anmeldung: 02.03.2005
(73) Patentinhaber: Grünenthal GmbH, 52078 Aachen (DE)
(72) Erfinder: HOLENZ, Jörg, 15023 Enhörna (SE); BUSCHMANN, Helmut, 08960 Sant Just Desvern Barcelona (ES)
(74) Vertreter: Kutzenberger, Helga
(86) Internationale Anmeldenummer: PCT/EP2003/005971
(87) Internationale Veröffentlichungsnummer: WO 2003/103650

(56) Entgegenhaltungen:
- WO-A-02/43715
- WO-A-02/066025
- WO-A-02/067651

## Beschreibung

Die Erfindung betrifft Wirkstoffsalze und Ester von 1-Dimethylamino-3-(3-methoxy-phenyl)-2-methyl-pentan-3-ol und 3-(3-Dimethylamino-1-ethyl-1-hydroxy-2-methyl-propyl)-phenol, Verfahren zu deren Herstellung, deren Verwendung zur Herstellung eines Arzneimittels zur Behandlung von vermehrtem Harndrang bzw. Harninkontinenz sowie entsprechende Arzneimittel und Verfahren zur Behandlung von vermehrtem Harndrang bzw. Harninkontinenz und Schmerz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z.B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskel der Harnröhre. Es treten Mischformen dieser Inkontinenzarten sowie die sogenannte Überflussinkontinenz z.B. bei benigner Prostatahyperplasie) oder Reflexinkontinenz (z. B. nach Rückenmarkschädigungen) auf. Näheres dazu findet sich bei Chutka, D. S. und Takahashi, P. Y., 1998, Drugs 560: 587-595

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A: J., 1998, Urology 51 (Suppl. 21): 43-47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol.

Einen genauen Einblick in die verwendeten Therapeutika und Therapiemethoden, insbesondere bezüglich der Antimuskarinika und anderer peripher wirkender Stoffe, gibt hier der Übersichtsartikel von K. E. Andersson et al. "The pharmacological treatment of urinary incontinence", BJU International (1999), 84, 923-947.

Auch bestimmte Diarylmethylpiperazine und -piperidine sind für diese Indikation in der WO 93/15062 beschrieben. Ebenso wurde für Tramadol ein positiver Effekt auf die Blasenfunktion in einem Rattenmodell rhythmischer Blasenkontraktionen nachgewiesen (Nipon-Shinyaku, WO 98/46216). Weiterhin gibt es in der Literatur Untersuchungen zur Charakterisierung der oioiden Nebenwirkung Harnretention, woraus sich einige Hinweise auf die Beeinflussung der Blasenfunktionen durch schwache Opioide wie Diphenoxylat (Fowler et al. 1987 J. Urol 138: 735-738) und Meperidin (Doyle and Briscoe, 1976 Br J Urol 48: 329-335), durch gemischte Opioidagonisten / - antagonisten wie Buprenorphin (Malinovsky et al., 1998, Anesth Analg 87:456-461; Drenger and Magora, 1989 Anesth Analg 69:348-353), Pentazocin (Shimizu et al. (2000) Br. J. Pharmacol. 131 (3): 610 - 616) und Nalbuphin (Malinovsky et al., 1998, a.a.O), sowie durch starke Opioide wie Morphin (Malinovsky et al., 1998 a.a.O; Kontani und Kawabata, (1988).; Jpn J Pharmacol. Sep;48(1):31) und Fentanyl (Malinovsky et al., 1998 a.a.O) ergeben. Allerdings erfolgten diese Untersuchungen zumeist in analgetisch wirksamen Konzentrationen.

Bei den hier in Frage kommenden Indikationen ist zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier noch mehr als bei Analgetika darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Aufgabe der vorliegenden Erfindung war es daher, Stoffe oder Stoffkombinationen aufzufinden, die zur Behandlung von vermehrtem Harndrang bzw. Harninkontinenz hilfreich sind und bei denen wirksamen Dosen bevorzugt gleichzeitig geringere Nebenwirkungen und/oder analgetische Wirkungen zeigen als aus dem Stand der Technik bekannt, insbesondere einen synergistischen Effekt zur Behandlung der Harninkontinenz zeigen.

Gegenstand der Erfindung ist daher ein Wirkstoffsalz von 1-Dimethylamino-3-(3-methoxy-phenyl)-2-methyl-pentan-3-ol gebildet aus 1-Dimethylamino-3-(3-methoxy-phenyl)-2-methyl-pentan-3-ol und einem Wirkstoff ausgewählt aus:
- Ibuprofen
- (S)(+) Ibuprofen
- (S)(+) Naproxen
- Diclofenac
- Acetylsalicylsäure
- Dipyron
- Indomethazin
- Ketoprofen
- Isoxicam
- Flurbiprofen
- Piroxicam oder
- Phenylbutazon
in Bezug auf 1-Dimethylamino-3-(3-methoxy-phenyl)-2-methyl-pentan-3-ol und/oder den Wirkstoff gegebenenfalls in Form einer Racemate, der reinen Stereoisomeren, insbesondere der Entantiomeren oder Diastereomeren, in einem beliebigen Mischungsverhältnis.

Dabei ist es besonders bevorzugt, wenn es sich um das Wirkstoffsalz von (+)-(2R,3R)-1-Dimethylamino-3-(3-methoxy-Phenyl)-2-methyl-pentan-3-ol handelt.

Ein weiterer Gegenstand der Erfindung ist ein Wirkstoffsalz von 3-(3-Dimethylamino-1-ethyl-t-hydroxy-2-methyl-propyl)-phenol gebildet aus 3-(3-Dimethylamino-1-ethyl-1-hydroxy-2-methyl-propyl)-phenol und einem Wirkstoff ausgewählt aus:
- lbuprofen
- (S)(+) lbuprofen
- (S)(+) Naproxen
- Diclofenac
- Acetylsalicylsäure
- Dipyron
- Indomethazin
- Ketoprofen
- lsoxicam
- Flurbiprofen
- Piroxicam oder
- Phenylbutazon

in Bezug auf 3-(3-Dimethylamino-1-ethyl-1-hydroxy-2-methyl-propyl)-phenol und/oder den Wirkstoff gegebenenfalls in Form der Racemate, der reinen Stereoisomeren, insbesondere Enantionmeren oder Diastereomeren, oder in Form von Mischungen der Stereoisomeren, insbesondere der Enantiomeren oder Diastereomeren, in einem beliebigen Mischungsverhältnis.

Dabei ist es besonders bevorzugt, wenn es sich um das Wirkstoffsalz von (+,-(2R,3R)-3-(3-Dimethylamino-1-ethyl-1-hydroxy-2-methyl-propyl)-phenol handelt.

Ein weiterer Gegenstand der Erfindung ist ein Ester von 3-(3-Dimethylamino-1-ethyl-1-hydroxy-2-methyl-propyl)-phenol.gemäß Formel 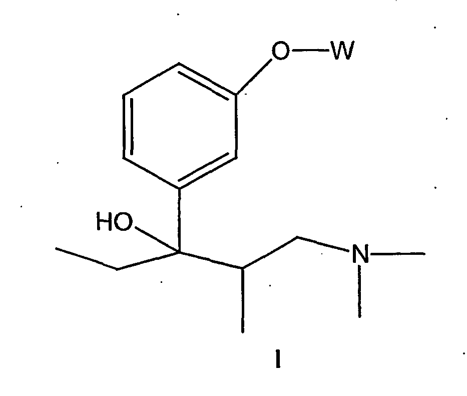 mit W ausgewählt aus dehydroxyliertem
- lbuprofen,
- (S)(+) Ibuprofen,
- (S)(+) Naproxen,
- Indomethazin,
- Diclofenac,
- Dipyron,
- Flurbiprofen,
- Ketoprofen oder
- Acetylsalicylsäure,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis, in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Dabei ist besonders bevorzugt, wenn der Ester gemäß Formel 1 in der Konformation der Formel 1b 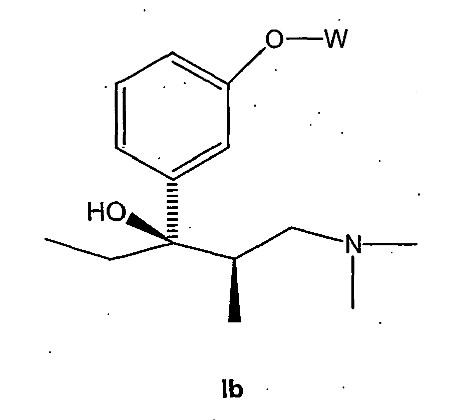 vorliegt.

Ein weiterer Gegenstand der Erfindung ist ein Ester gemäß Formel 11 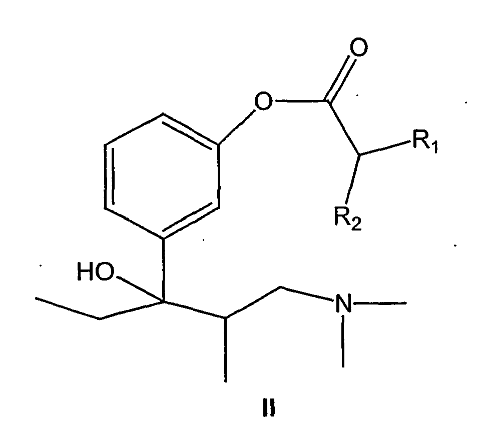 mit R₁ ausgewählt aus Aryl und Heteroaryl, jeweils substituiert oder unsubstituiert, und R₂ ausgewählt aus H oder R₁₋₃-Alkyl oder ungesättigt, unsubstituiert oder ein- oder mehrfach substituiert, gegebenenfalls in Form der Racemate, der reinen Stereoisomeren, insbesondere Enantiomeren oder Diastereomeren, oder in Form von Mischungen der Stereoisomeren , insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Für erfindungsgemäße Ester gemäß Formel II ist es besonders bevorzugt, wenn der Ester in der Konformation IIb 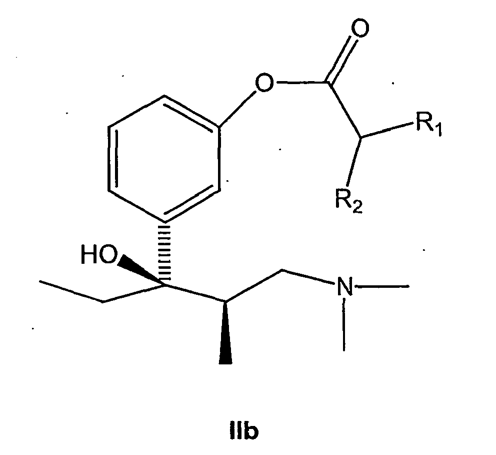 vorliegt.

Für erfindungsgemäße Ester gemäß Formel II oder IIb ist es besonders bevorzugt, wenn R₁ ausgewählt ist aus

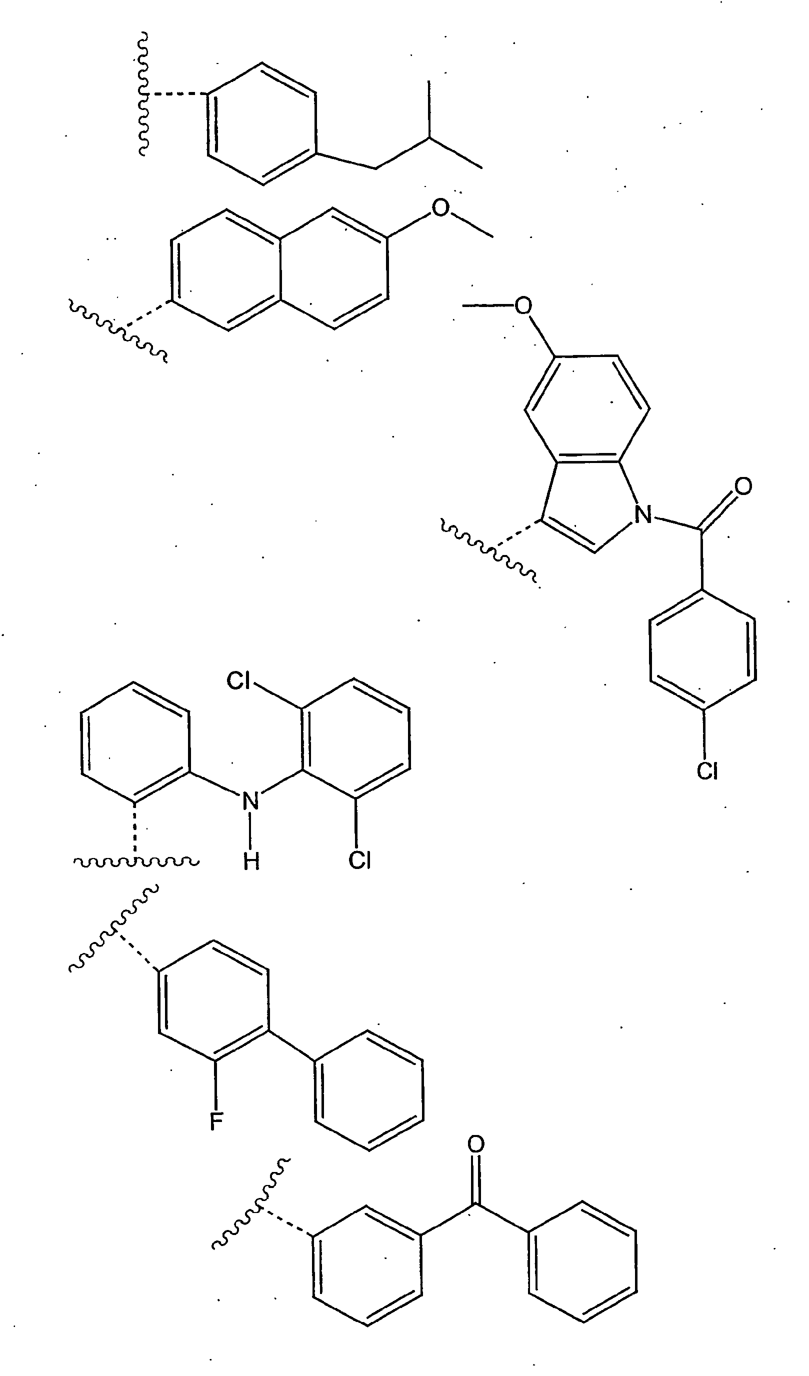

Für efindungsgemäße Ester gemäß Formel 11 oder IIb ist es besonders bevorzugt, wenn R₂ ausgewählt ist aus H oder CH₃.

Ein weiterer Gegenstand der Erfindung ist ein Ester gemäß Formel III 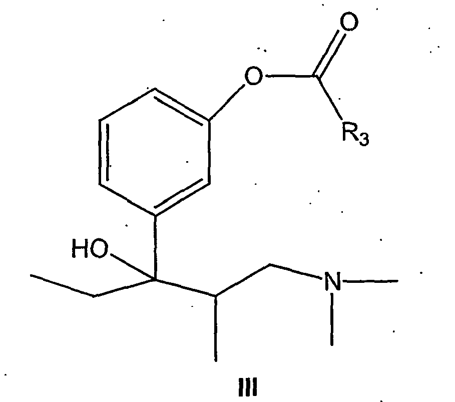 mit R₃ ausgewählt aus Aryl und Heteroaryl, jeweils substituiert oder unsubstituiert,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Für erfindungsgemäße Ester gemäß Formel III ist es besonders bevorzugt, wenn der Ester in der Konformation IIIb 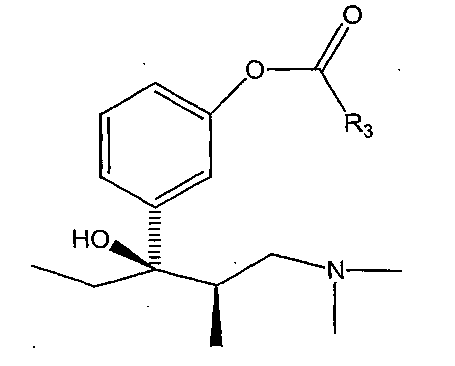 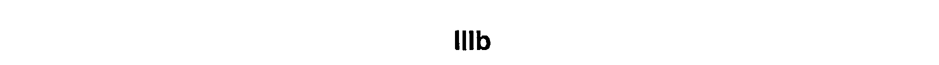 vorliegt.

Für erfindungsgemäße Ester gemäß Formel III ist es besonders bevorzugt, wenn R₃ ausgewählt aus

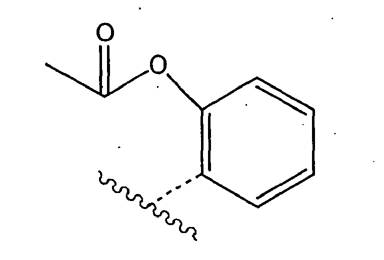

Ein weiterer Gegenstand der Erfindung ist ein Ester gemäß Formel IV

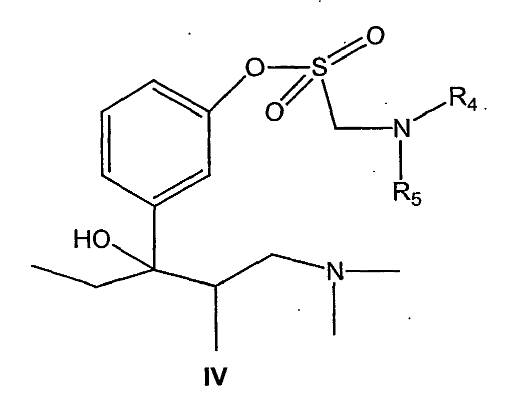

Mit R₄ ausgewählt aus Aryl und Heteroaryl, jeweils substituiert oder unsubstituiert, und R₅ ausgewählt aus H oder C₁₋₃-Alkyl, gesättigt oder ungesättigt, unsubstituiert oder ein- oder mehrfach substituiert, gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Sale, insbesondere der physiologisch verträglichen Salze, oder in Form ihrer Solvate, insbesondere der Hydrate.

Für erfindungsgemäße Ester gemäß Formel IV ist es besonders bevorzugt, wenn der Ester in der Konformation IVb 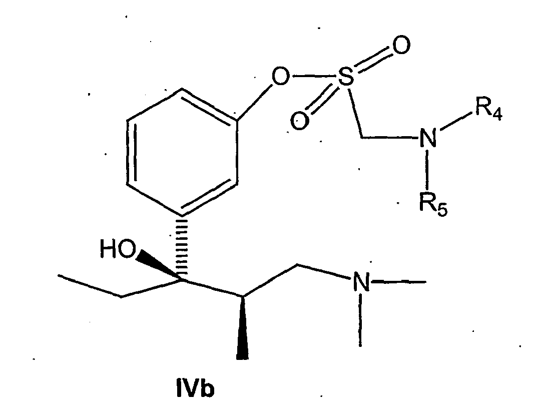 vorliegt.

Für erfindungsgemäße Ester gemäß Formel IV oder IVb ist es besonders bevorzugt, wenn R₄ ausgewählt ist aus

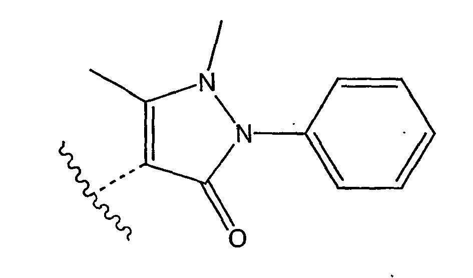

Für erfindungsgemäße Ester gemäß Formel IV oder lVb ist es besonders bevorzugt, wenn R₅ ausgewählt ist aus H oder CH₃.

Überraschenderweise hatte sich herausgestellt, daß die genannten erfindungsgemäßen Substanzen bestimmte physiologische Parameter, die bei vermehrtem Harndrang bzw. Harninkontinenz von Bedeutung sind, deutlich positiv beeinflussen, so entweder den Schwellendruck, das Interkontraktionsintervall bzw. die Verringerung der rhythmischen Blasenkontraktionen und/oder die Blasenkapazität. Jede einzelne dieser Veränderungen kann eine deutliche Erleichterung im symptomatischen Bild von betroffenen Patienten bedeuten.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3- oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfasst der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methyl-propyl, 2-Methyl-propyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCI₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂CH₂-CH₂-CH₂-CH₂- und CH₂-CH₂-CH₂-CH₂-CH₂-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄-₅ ist -CH₂-CH₂-CH₂-CH₂- und -CH₂-CH₂-CH₂-CH₂-CH₂- zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-[1,2,5]thiadiazol, Benzothiazol, Indol, Benzotriazol, Benzodioxolan, Benzodioxan, Carbazol, und Chinazolin aufgeführt.

Dabei versteht man im Zusammenhang mit Aryl und Heteroaryl unter substituiert die Substitution des Aryls oder Heteroaryls mit R²², OR²² einem Halogen, vorzugsweise F und/oder Cl, einem CF₃, einem CN, einem NO₂, einem NR²³R²⁴, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²² für H, einen C₁₋₁₀-Alkyl-, vorzugsweise einen C₁₋₆-Alkyl-, einen Aryl- oder Heteroaryl- oder für einen über eine C₁₋₃-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen,
die Reste R²³ und R²⁴, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²³ und R²⁴ bedeuten zusammen CH₂CH₂OCH₂CH₂, CH₂CH₂NR²⁵CH₂CH₂ oder (CH₂)₃₋₆, und
der Rest R²⁵ für H, einen C₁₋₁₀-Alkyl-, vorzugsweise einen C₁₋₆-Alkyl-, einen Aryl-, oder Heteroaryl- Rest oder für einen über eine C₁₋₃-Alkyl, gesättigt oder ungesättigt, oder eine C₁₋₃ Alkylen-Gruppe gebundenen Aryl- oder Heteroaryl-Rest, wobei diese Aryl und Heteroarylreste nicht selbst mit Aryl- oder Heteroaryl-Resten substituiert sein dürfen. Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder ein mit einer physiologisch verträglichen Säure oder einem physiologisch verträglichen Kation gebildetes Salz.

Unter dem Begriff des physiologisch verträglichen Salzes mit Kationen oder Basen versteht man im Sinne dieser Erfindung Salze mindestens einer der erfindungsgemäßen Verbindungen - meist, beispielsweise am Stickstoff protoniert - als Kation mit mindestens einem Anion, die physiologisch insbesondere bei Anwendung i Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1λ⁶-benzo[d]isothiazol-3-on (Saccharinsäure) Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz,

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfdnsäure, Nicotinsäure, 2-, 3- oder 4-Aminobenzoesäure, 2,4,6-Trimethyl-benzoesäure, a-Liporisäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprontonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder im Säugetier - verträglich ist. Besonders bevorzugt sind Salze der Alkali- und Erdalkalimetalle aber auch NH₄⁺, insbesondere aber (Mono-) oder (Di-) Natrium-, (Mono-) oder (Di-) Kalium-, Magnesium- oder Calzium Salze. Besonders bevorzugt ist das Hydrochlorid.

1-Dimethylamino-3-(3-methoxy-phenyl)-2-methyl-pentan-3-ol, (2RS,3RS)-1-Dimethylamino-3-(3-methoxy-phenyl)-2-methyl-pentan-3-ol, (+)-(2R,3R)-1-Dimethylamino-3-(3-methoxy-phenyl)-2-methyl-pentan-3-ol, (-)-(2S,3S)-1-Dimethylamino-3-(3-methoxy-phenyl)-2-methyl-pentan-3-ol, 3-(3-Dimethylamino-1-ethyl-1-hydroxy-2-methyl-propyl)-phenol, (2RS,3RS)-3-(3-Dimethylamino-1-ethyl-1-hydroxy-2-methyl-propyl)-phenol, (+)-(2R,3R)-3-(3-Dimethylamino-1-ethyl-1-hydroxy-2-methyl-propyl)-phenol, (-)-(2S,3S)-3-(3-Dimethylamino-1-ethyl-1-hydroxy-2-methyl-propyl)-phenol und deren Herstellung sind aus der DE 44 26 245 A1 bzw. der US 6,248,737 B1 bekannt. Die anderen Wirkstoffe, mit denen diese Verbindungen reagiert werden, sind bekannte Analgetika, die käuflich zu erwerben sind und deren Herstellung dem Fachmann hinlänglich bekannt sind.

Die erfindungsgemäßen Ester und Salze sind toxikologisch unbedenklich und medizinisch wirksam.

Ein weiterer Gegenstand der Erfindung ist daher ein Arzneimittel, vorzugsweise zur Behandlung von vermehrtem Harndrang bzw. Harninkontinenz, enthaltend mindestens ein erfindungsgemäßes Wirkstoffsalz oder einen erfindungsgemäßen Ester sowie gegebenenfalls geeignete Zusatz- und/oder Hilfsstoffe.
Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht wenigstens einer Verbindung der Formel 1 appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, ',gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.
So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzung zu bilden, die Wirkstoff in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/ode.r Celluloseacetat.

Auch wenn die erfindungsgemäßen'Arzneimittel lediglich geringe Nebenwirkungen zeigen, kann es beispielsweise zur Vermeidung von bestimmten Formen der Abhängigkeit von Vorteil sein, neben den erfindungsgemäßen Verbindungen auch Morphinantagonisten, insbesondere Naloxon, Naltrexon und/oder Levallorphan, zu verwenden.

Die Verbindungen sind sehr gut zur Behandlung von vermehrtem Harndrang bzw Harninkontinenz insbesondere bei Stressinkontinenz und Dranginkontinenz einsetzbar. Entsprechend ist ein weiterer Gegenstand der Erfindung die Verwendung eines erfindungsgemäßen Wirkstoffsalzes oder eines erfindungsgemäßen Esters zur Herstellung eines Arzneimittels zur Behandlung vom vermehrtem Harndrang bzw. Harninkontinenz.

Die Verbindungen sind weiter hervorragende Analgetika, entsprechend ist ein weiterer Gegenstand der Erfindung die Verwendung eines erfindungsgemäßen Wirkstoffsalzes oder eines erfindungsgemäßen Esters zur Herstellung eines Arzneimittels zur Behandlung von Schmerz insbesondere chronischem, biszeralem, neuropathischem oder akutem Schmerz oder Entzündungs-Schmerz.

Weiter betrifft die Erfindung auch ein Verfahren zur Behandlung von vermehrtem Harndrang bzw Harninkontinenz bzw. Schmerz, bei dem erfindungsgemäße Verbindungen verwendet werden.

Die folgenden Beispiele sollen die Erfindung erläutern, ohne dass der Gegenstand der Erfindung darauf beschränkt wäre.

### Beispiele

### Beispiel 1: Liste der getesteten Substanzen:

Es folgt eine Liste der auf ihre Wirksamkeit getesteten Verbindungen:

| Name | Verbdg. Nr. |
|---|---|
| (2RS,3RS)-1- Dimethylamino-3-(3-methoxy-phenyl)-2-methylpentan-3-ol, Hydrochlorid , | 1 |
| (+)-(2R,3R)-1- Dimethylamino-3-(3-methoxy-phenyl)-2-methylpentan-3-ol, Hydrochlorid | 2 |
| (-)-(2S,3S)-1- Dimethylamino-3-(3-methoxy-phenyl)-2-methylpentan-3-ol, Hydrochlorid | 21 |
| (2RS,3RS)-3-(3- Dimethylamino-1-ethyl-1-hydroxy-2-methylpropyl)-phenol, Hydrochlorid | 7 |

### Beispiel 2: Testsystem Cystometrie an der wachen naiven Ratte

Es wurden cystometrische Untersuchungen an naiven, weiblichen Sprague-Dawley-Ratten nach der Methode von lshizuka et al. ((1997), Naunyn-Schmiedeberg's Arch. Pharmacol. 355: 787-793) durchgeführt. Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet. Nach einer Stabilisierungsphase wurde eine 20minütige Phase aufgezeichnet, die durch normale, reproduzierbare Miktionszyklen gekennzeichnet war. Es wurden unter anderem die folgenden Parameter bestimmt:
- Schwellendruch (threshold pressure TP, Blasendruck unmittelbar vor Miktiön),
- Blasenkapazität (bladder capacity BC, Restvolumen nach vorhergehender Miktion plus Volumen der infundierten Lösung während der Füllungsphase),
- Interkontraktionsintervall (inter-contraction interval (ICI), das Zeitintervall zwischen den Miktionen).

Eine Erhöhung des Schwellendrucks (TP) zeigt eine wichtige therapeutische Wirkung bei einer der erfindungsgemäßen Indikationen an. Auch das fnterkontraktionsintervall (ICI) ist ein wichtiger Parameter zur Messung der physiologischen Wirksamkeit eines Stoffes in der Behandlung der Harninkontinenz, ebenso wie die Blasenkapazität (BC). Dabei ist es für eine Wirksamkeit aufgrund der sehr heterogenen Ursachen für die Symptomatik dieser Erkrankungsbilder nicht nötig, alle drei Parameter positiv zu beeinflussen. Es genügt daher völlig, wenn nur in einem dieser Parameter eine positive Wirkung festzustellen ist, um in der Harninkontinenz oder vermehrtem Harndrang einsetzbar zu sein.

Nach der Aufzeichnung von drei reproduzierbaren Miktionszyklen als Vorwert, wurden die Testsubstanzen 1 (1,0 mg/kg), 2 (0,1; 0,3 und 0,5 mg/kg), 21 (0,5 mg/kg) und 7 (0,3 mg/kg) im Vehikel = 0,9 % NaCl i. v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt. (Tabelle 1).

**Tabelle 1: Beeinflussung der cystometrischen Parameter durch die Testsubstanzen (Veränderung zum Vorwert [%]), n entspricht der Anzahl der Versuchstiere, Signifikanz (Student T-Test): * p < 0.005; * p < 1.01; *** p < 0.001.**

| Verbindung | TP Theshold pressure | BC Bladder capcity | ICl Intercontraction interval |
|---|---|---|---|
| **1** | | | |
| 0,1 mg/kg iv (n=9) | +94% ** | +31 % ** | +42% |
| **2** | | | |
| 0,1 mg/kg iv (n=5) | +28,5% ** | +7,8% ** | +15,6% |
| 0,3 mg/kg iv (n=8) | +122% ** . | +33% ** | +28%* |
| 0,5 mg/kg iv (n=9) | +77,5% ** | +20,6% ** | ±28,6% ** |
| **21** | | | |
| 0,5 mg/kg iv (n=8) | -1.1%** | +3%** | +10% |
| **7** | | | |
| 0,3 mg/kg iv (n=7) | +95% ** | +32% ** | +2%* |

Die untersuchten Substanzen zeigten eine positive Wirkung auf die Blasenregulation und sind somit geeignet zur Behandlung der Harninkontinenz.

Unter anderem zeigt sich, dass von den Enantionmeren der racemischen Verbindung 1 (+)-Enantiomere (Verbindung 2) sehr wirksam ist (und damit eine besonders bevorzugte Verbindung dieser Erfindung ist), während das (-)-Enantiomere (Verbindung 21) nicht so stark zur Wirkung beisteuert.

### Die folgenden Verbindungen (Ester und Salze) wurden synthetisiert:

Generell gilt, das im folgenden
(+) (2R,3R)-3-(3- Dimethylamino-1-ethyl-1-hydroxy-2-methyl-propyl)-phenol als **M1** und
(+)-(2R,3R)-1- Dimethylamino-3-(3-methoxy-phenyl)-2-methyl-pentan-3-ol als **M0** bezeichnet wird

### BEISPIEL 30: ALLGEMEINES VERFAHREN ZUR HERSTELLUNG DER ESTER

### Reaktionsgleichung:

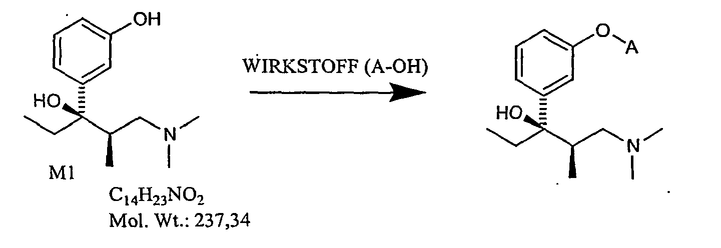

| **Ansatzgröße:** | | | |
|---|---|---|---|
| X mmol | M1 | X' mg | 1, 00eq |
| Y mmol | A-OH | Y mg | 0,95 eq |
| Z mmol | DCC (N,N-Dicyclohexylcarbodiimid) | Z mg | 1,40 eq |
| V mmol | DMAP (N,N-Dimethylaminopyridin) | V' mg | 0,086 eq |
| Dichlormethan | | | |

| | | | |
|---|---|---|---|
| **Apparatur:** Dreihalskolben (DHK), Magnetrührer, Stickstoff, Eisbad | | | |
| **Durchführung/Reaktionsbedingungen:** Die Apparatur wird aufgeheizt (Molsieb schon vorgelegt). Die Säure und das M1 werden abgewogen und in Dichlormethan gelöst zugegeben. Unter Rühren DMAP zugeben. Mit einem Eisbad auf 0° C kühlen und das DCC in Dichlormethan gelöst zugeben. Im Eisbad langsam auftauen lassen und 2 Tage rühren. | | | |
| | | | |
| Der entstandene Niederschlag und die Molsiebe werden abfiltriert (N,N-Dicyclohexylharnstoff) und man wäscht mit Dichlormethan nach. Das Filtrat wird am Rotationsverdampfer bei RT bis zur Trockene eingeengt. Der Rückstand wird in Dichlormethan aufgenommen und erneut filtriert. | | | |
| **Aufarbeitung:** Das Filtrat wird mit einer 0,5 molaren HCL-Lösung gewaschen. Phasen trennen und die HCL-Phase noch mal mit Dichlormethan ausschütteln. Die org. Phase mit 10%iger Nariumhydrogencarbonatlösung ausschütten, die wässrige Phase erneut mit Dichlormethan waschen. Die vereinigten org. Phasen über Magnesiumsulfat trocknen und am Rotationsverdampfer bei RT einengen. Mindestens 1 h trockenziehen | | | |

| **Aufreinigung durch Säulenchromatographie an Kieselgel** | | | |
|---|---|---|---|
| **HC1-Fällung** | | | |
| **Ansatz:** | | | |
| Substanz | | 1,0 eq | |
| Trimethylchlorsilan | | 1,2 eq | |
| | | | Ether |
| | | | MEK (Methyl-ethyl-keton bzw. 2-Butanon) |
| | | | |
| Die Base in wenig MEK lösen und mit Ether auffüllen bis sich die Lösung leicht trübt. Trimethylchlorsilan zugeben und 3 Tage bei RT (Raumtemperatur) rühren lassen. | | | |
| Der Hydrochloridniederschlag wird abfiltriert, mit Ether gewaschen und am Rotationsverdampfer getrocknet. | | | |

### Beispiel 31

### Ester mit Fluorbiprofen

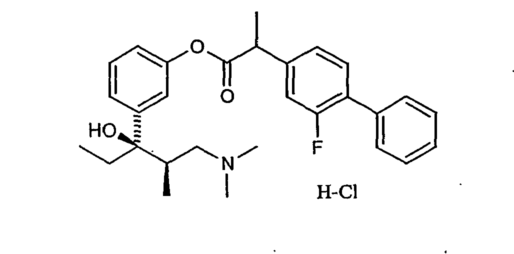

| **Reaktionsgleichung:** | | | |
|---|---|---|---|
| 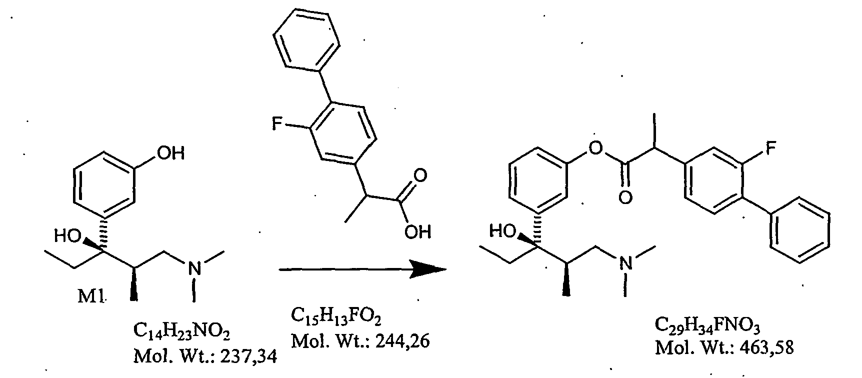 | | | |

| **Ansatzgröße:** | | | |
|---|---|---|---|
| 2,11 mmol | M1 | 500 mg | |
| 2,00 mmol | Fluorbiprofen | 488 mg | 0,95 eq |
| 2,95 mmol | DCC | 592 mg | 1,40 eq |
| 0,18 mmol | DMAP | 22 mg | 0,086 |
| | | | |
| | Dichlormetan | 25 ml | |
| **Apparatur:** 100 ml DHK, Stickstoff, Magnetrührer, Eisbad | | | |
| **Durchführung/Reaktionsbedingungen:** Die Apparatur wird aufgeheizt (Molsieb schon vorgelegt). Die Säure und das M1 werden abgewogen und in Dichlormethan gelöst zugegeben. Unter Rühren DMAP zugeben. Mit einem Eisbad auf 0°C kühlen und das DCC in Dichlormethan gelöst zugeben. Im Eisbad langsam auftauen lassen und 2 Tage rühren. | | | |
| Der entstandene Niederschlag und die Molsiebe werden abfiltriert (N,N-Dicyclohexylharnstoff) und man wäscht mit 5 mL Dichlormetan nach. Das Filtrat wird am Rotationsverdampfer bei. RT bis zur Trockene eingeengt. Der Rückstand wird in 5 mL Dichlormetan aufgenommen und erneut gefiltert. | | | |
| **Aufarbeitung:** Das Filtrat wird mit einer 0,5 molaren HCL-Lösung gewaschen. Phasen trennen und die HCL-Phase noch mal mit Dichlormethan ausschütteln. Die org. Phase mit 10%iger Natriumhydrogencarbonatlösung ausschütten, die wässrige Phase erneut mit Dichlormethan waschen. Die vereinigten org. Phasen über Magnesiumsulfat trocknen und am Rotationsverdampfer bei RT einengen. Mindestens 1 h trockenziehen | | | |
| **Auswaage: 0,95 g** | | **97% d. Theo** | |

| **Aufreinigung durch Säulenchromatographie** | | | |
|---|---|---|---|
| **Säule:** | Länge: | 15 cm | |
| | Durchmesser: | 6 cm | |
| | Kieselgel: | sauer | |
| **Fließmittel: Essigester / Methanol 5:1** | | | |
| **Zu trennende Substanzmenge: 0,95 g** | | | |
| **Fraktionsschritte 100 ml** | | | **Vorlauf: 200 ml** |

| **Fraktionen:** | | | |
|---|---|---|---|
| Fr1 | VI bis 2 = | | |
| Fr2 | 3 bis 4 = 360 mg | | |
| **DC:** | Fließmittel: | EE/ MeOH 5:1 | |
| | Detektion: | UV /J₂ | |
| | Vergleichssubstanzen: | Substanz-Rohgemisch | |

### HCI-Fällung:

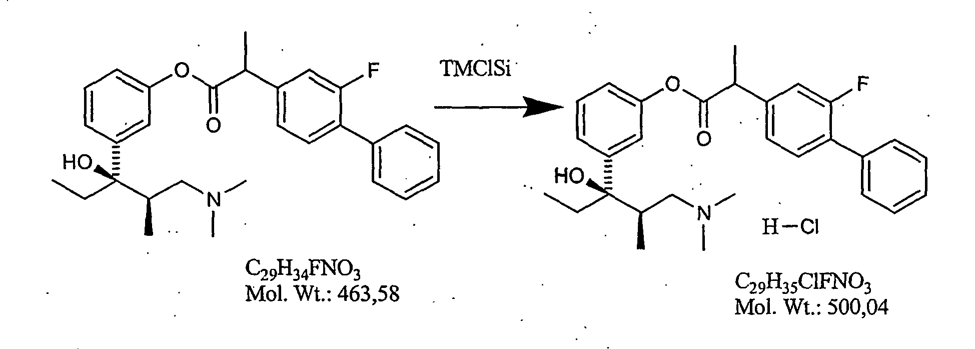

Die Base in wenig MEK lösen und mit Ether auffüllen, bis sich die Lösung leicht trübt. Trimethylchlorsilan zugeben und 3 Tage bei RT rühren lassen. Der farblose Hydrochloridniederschlag wird abfiltriert, mit Ether gewaschen und am Rotationsverdampfer getrocknet.
- **Auswage:**: **270 mg**
- **Analytik:**: LC-MS
¹H NMR
Smp. (Schmelzpunkt): 128,3 °C

### Beispiel 32:

### Ester mit Diclofenac

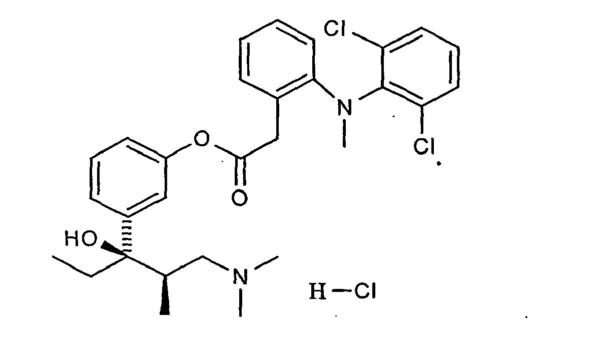

| **Reaktionsgleichung:** | | | |
|---|---|---|---|
| 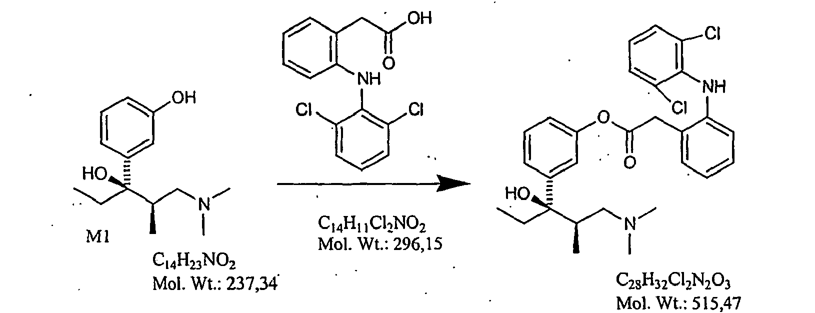 | | | |

| **Ansatzgröße:** | | | |
|---|---|---|---|
| 4,21 mmol | M1 | 1000 mg | |
| 4,00 mmol | Diclofenac | 1185 mg | 0,95 eq |
| 5,89 mmol | DCC | 1180 mg | 1,40 eq |
| 0,36 mmol | DMAP | 40 mg | 0,086 eq |
| | Dichlormethan | 25 ml | |
| **Apparatur:** 100 ml DHK, Magnetrührer, Stickstoff | | | |
| **Durchführung/Reaktionsbedingungen:** Molsieb in die Apparatur einfüllen und ausheizen. Mit Stickstoff belüften. M1 und Säure in Dichlormethan lösen und einfüllen. Unter Rühren DMAP und DCC in Dichlormethan gelöst zugeben. Über Nacht rühren lassen. | | | |
| | | | |
| DC-Kontrolle mit Essigester / Methanol 5:1 => neuer Fleck oberhalb von M1 | | | |
| | | | |
| Der entstandene Niederschlag und die Molsiebe werden abfiltriert (N,N-Dicyclohexylharnstoff) und man wäscht mit 5 mL Dichlormethan nach. Das Filtrat wird am Rotationsverdampfer bei RT bis zur Trockene eingeengt. Der Rückstand wird in 5 mL Dichlormethan aufgenommen. | | | |
| **Aufarbeitung:** Das Filtrat wird mit einer 0,5 molaren HCL-Lösung gewaschen. Phasen trennen und die HCL-Phase noch mal mit Dichlormethan ausschütteln. Die org. Phase mit 10%iger Natriumhydrogencarbonatlösung ausschütteln, die wässrige Phase erneut mit Dichlormethan waschen. Die vereinigten org. Phasen über Magnesiumsulfat trocknen und am Rotationverdampfer bei RT einengen. 1 h trockenziehen. | | | |
| **Auswaage:** 2,48 g | 114% d. Theo. | | |
| **Optik:** orange, klar, klebrig wie Honig | | | |
| **Analytik:** | LC-MS | | |
| | ¹H NMR | | |
| **DC-Kontrolle:** | Essigester / Methanol 5:1 + Ammoniak | | |

| **Aufreinigung über Chromatographie:** | | | |
|---|---|---|---|
| **Säule:** | Länge: | 20 cm | |
| | Durchmesser: | 6 cm | |
| | Kieselgel: | sauer | |
| **Fließmittel: Essigester/Methanol 10:1 + Ammoniak 0,05%** | | | |
| **Zu trennende Substanzmenge: 2,48 g** | | | |
| **Fraktionsschritte:** | 50 ml | **Vorlauf:** | |

| **Fraktionen** | | | |
|---|---|---|---|
| Fr1 | VL bis 6 = | | |
| Fr2 | 7 bis 9 = 930 mg | | |
| Fr3 | 10 bis 15 = 460 mg | | |
| **Fr4** | **NL =** | | |
| **DC:** Fließmittel: Essigester/Methanol 10:1 + Ammoniak 0,05% Ammoniak | | | |
| | Detektion: UV / Jod | | |
| | Vergleichssubstanzen: Substanz-Rohgemisch | | |

### HCI-Fällung:

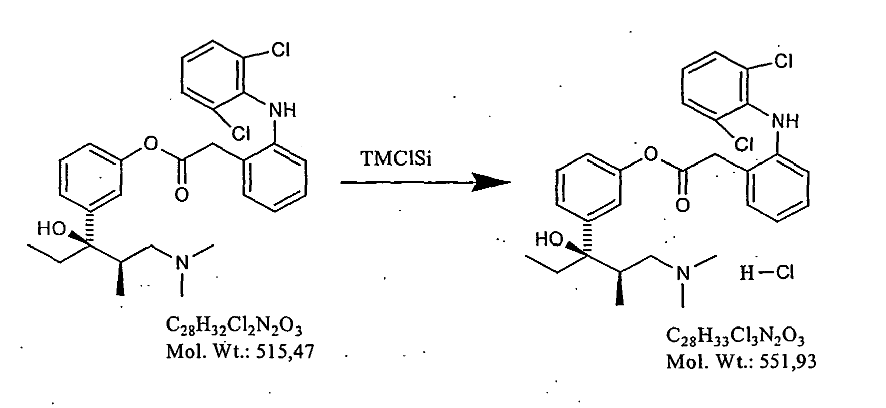

| **Ansatz 1:** | | |
|---|---|---|
| 1,8 mmol Substanz | 930 mg | |
| 2,2 mmol Trimethylchlorsilan. | 278 µl | 1,2 eq |

Ether
Aceton

Die Base in Ether aufnehmen und mit wenig Tropfen Aceton lösen. Unter Eiskühlung mit Trimethylchlorsilan versetzen. Sofort fällt ein farbloser Niederschlag aus. Über Nacht rühren lassen.
- **Auswaage:**: **680 mg**
- **Analytik:**: LC-MS ¹H NMR FR2
Smp.: 111,9 °C

Die folgenden Ester der Beispiele 33 bis 39 wurden wie vorhergehend beschrieben (s. Beispiel 30) hergestellt:

### Beispiel 33:

### Ester mit Ibuprofen

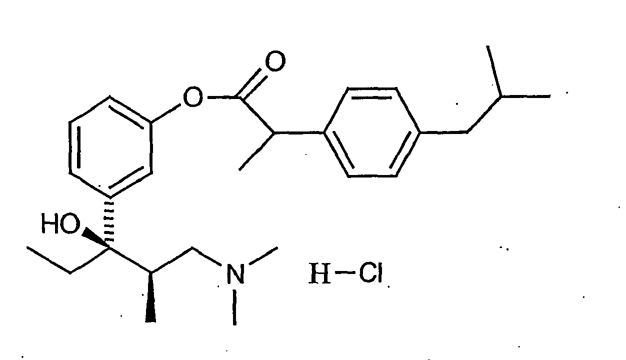

### Beispiel 34:

### Ester mit (S)(+)-Ibuprofen

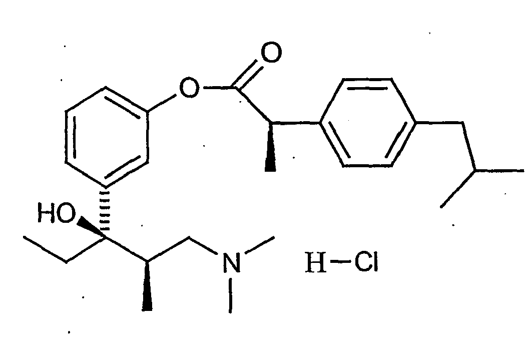

### Beispiel 35:

### Ester mit (S)(+)-Naproxen

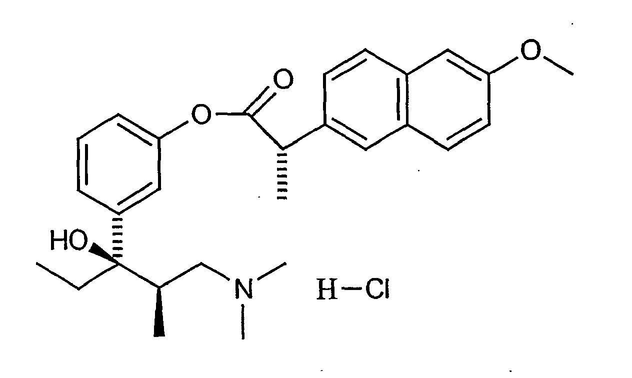

### Beispiel 36:

### Ester mit Indomethazin

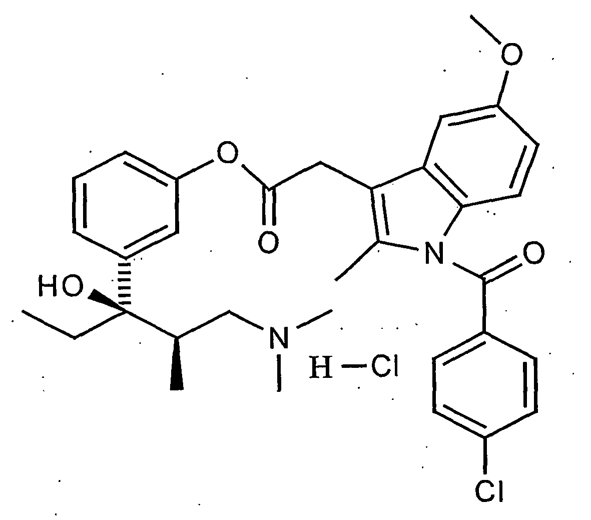

### Beispiel 37:

### Ester mit Dipyron

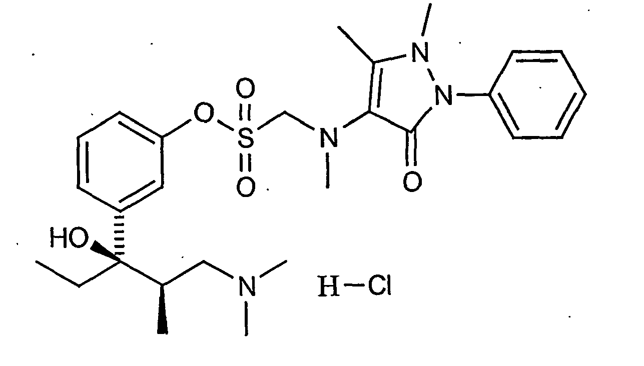

### Beispiel 38:

### Ester mit Ketoprofen

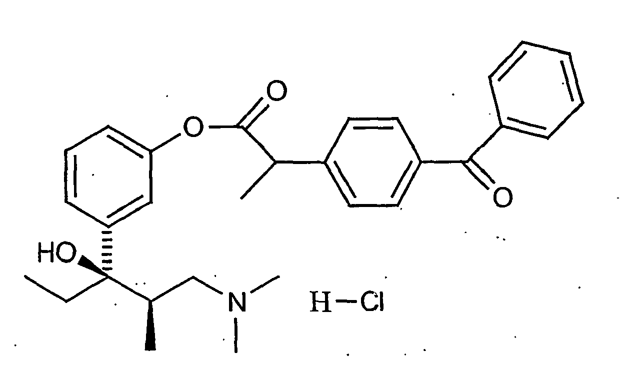

### Beispiel 39:

### Ester mit Acetylsalicylsäure

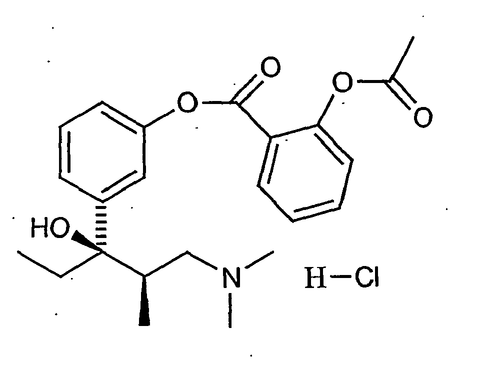

### BEISPIEL 40:

### ALLGEMEINES VERFAHREN ZUR HERSTELLUNG DER SALZE VON M1

| **Reaktionsgleichung** | |
|---|---|
| 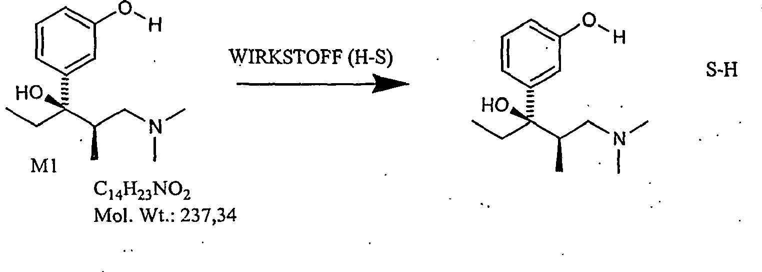 | |

| **Ansatzgröße:** | |
|---|---|
| x mmol M1 | z mg |
| x mmol S | y mg |
| Aceton | |
| **Apparatur:** 50 ml EHK, Rückflusskühler, Magnetrührer | |
| **Durchführung/Reaktionsbedingungen:** Base und Säure einwiegen und in so wenig wie möglich Aceton lösen. Bei RT bis ca. 40°C rühren lassen. Gegebenenfalls Lösungsmittel einengen. | |

### Beispiel 41

### Salz von M1 mit (s)(+) lbuprofen

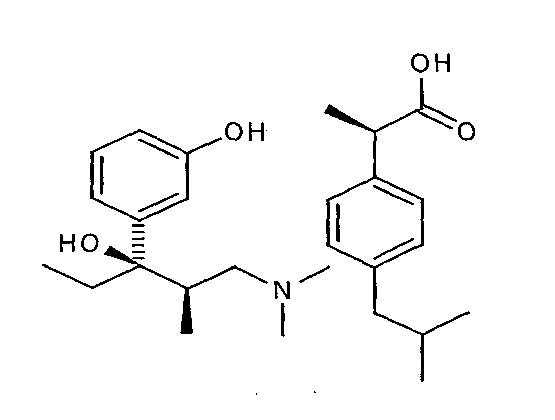

| **Reaktionsgleichung:** | |
|---|---|
| 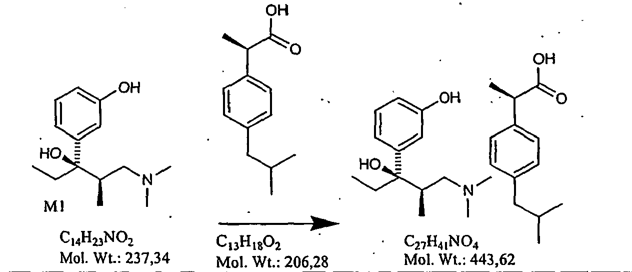 | |

| **Ansatzgröße:** | |
|---|---|
| 0,84 mmol M1 | 200 mg |
| 0,84 mmol (s)(+) lbuprofen | 173 mg |
| | |
| Aceton | |
| **Apparatur:** 50 ml EHK, Rückflusskühler, Magnetrührer | |
| **Durchführung/Reaktionsbedingungen:** Base und Säure einwiegen und in so wenig wie möglich Aceton lösen. Über Nacht bei RT rühren lassen. | |
| Es ist kein Niederschlag aufgefallen. Die Lösung auf 35-40°C erwärmen und 4h bei dieser Temperatur rühren lassen. Nach Einengen (Rotationsverdampfer) langsam auf RT abkühlen lassen. Es fällt ein farbloser Niederschlag | |
| **Auswaage:** 360 mg | 97% der Theo.f |
| **Analytik:** | ¹H NMR |

### Beispiel 42

### Salz von M1 mit Flurbiprofen

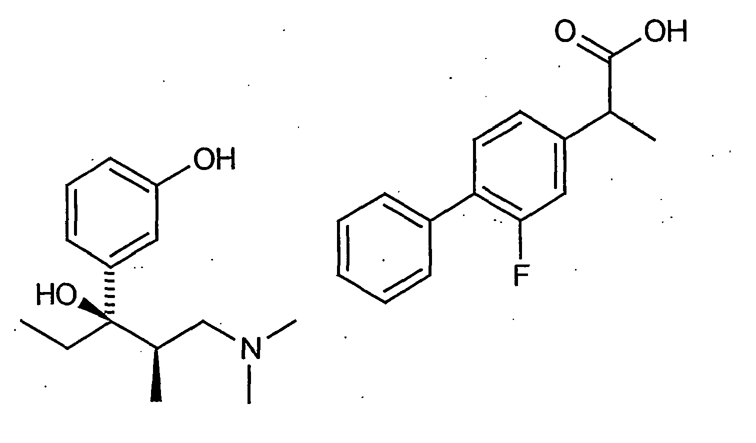

| **Reaktionsgleichung:** | |
|---|---|
| 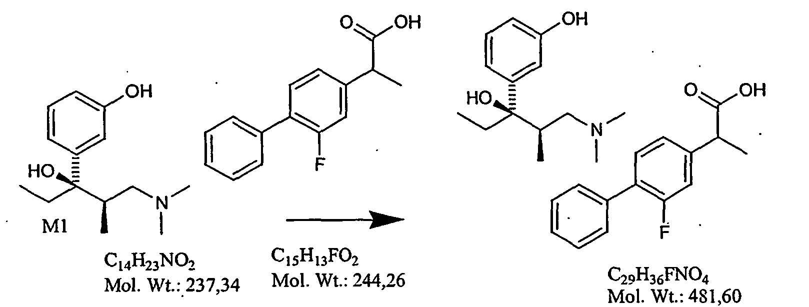 | |

| **Ansatzgröße:** | |
|---|---|
| 0,88 mmol M1 | 210 mg |
| 0,88 mmol Flurbiprofen | 215 mg |
| | |
| Aceton | |
| **Apparatur:** 50 ml EHK, Magnetrührer, Kühler | |
| **Durchführung/Reaktionsbedingungen:** Base und Säure einwiegen und in Aceton lösen. Die Lösung 5h bei 35-40°C rühren lassen. Über Nacht bei RT weiterrühren. | |
| **Aufarbeitung:** Nach Einengen fällt ein heller Feststoff. | |
| **Auswaage:** 420 mg | 99% der Theorie |
| **Analytik:** ¹H NMR | Smp.: 64,9 °C |

**Die folgenden Beispiele 43 bis 52 wurden wie vorhergehend beschrieben (s. Beispiel 40) hergestellt:**

### Beispiel 43

### Salz von M1 mit Ibuprofen

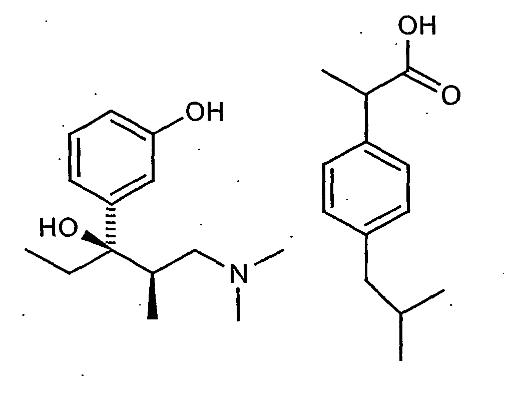

### Beispiel 44

### Salz von M1 mit (S)(+) Naproxen

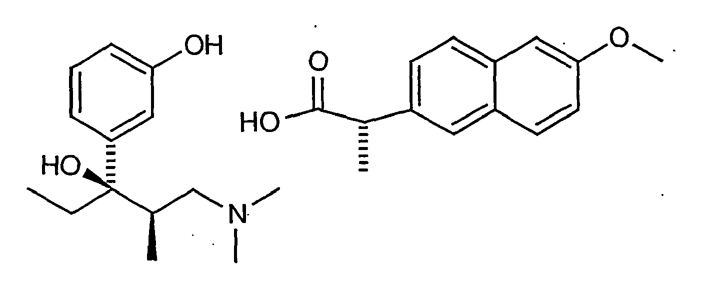

### Beispiel 45

### Salz von M1 mit Diclofenac

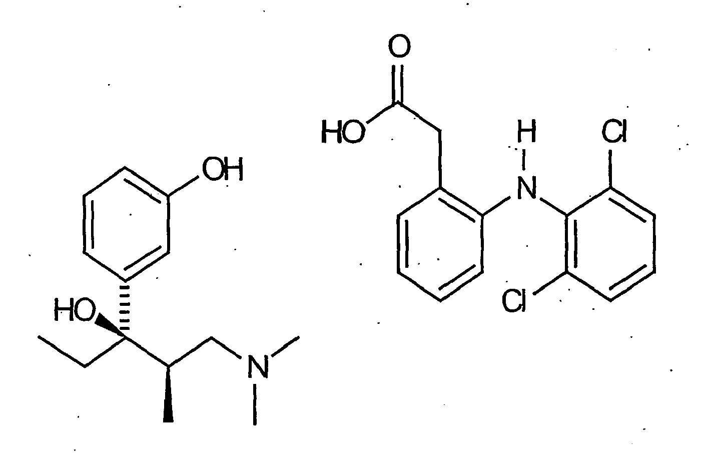

### Beispiel 46

### Salz von M1 mit Acetylsalicylsäure

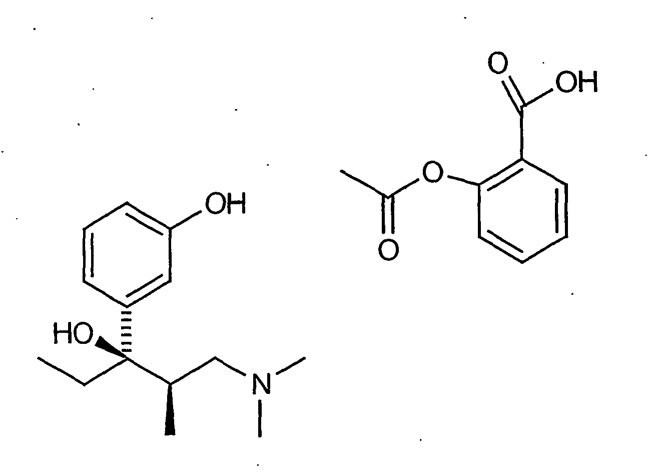

### Beispiel 47

### Salz von M1 mit Dipyron

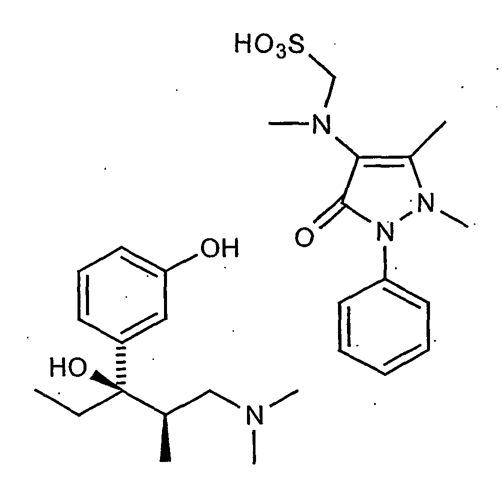

### Beispiel 48

### Salz von M1 mit Indomethazin

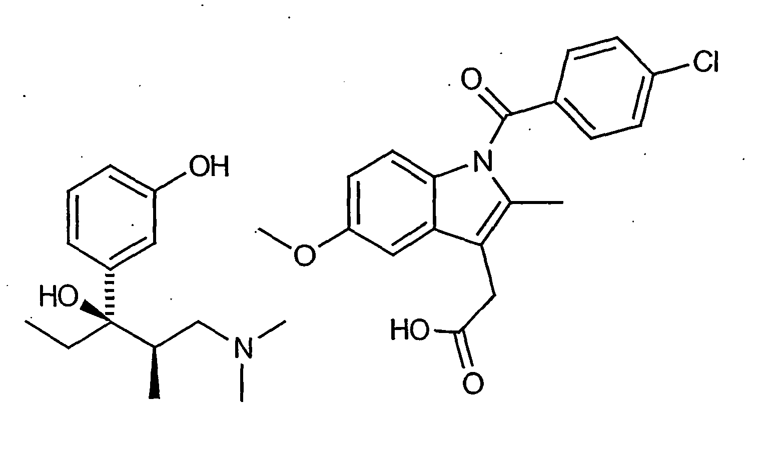

### Beispiel 49

### Salz von M1 mit Ketoprofen

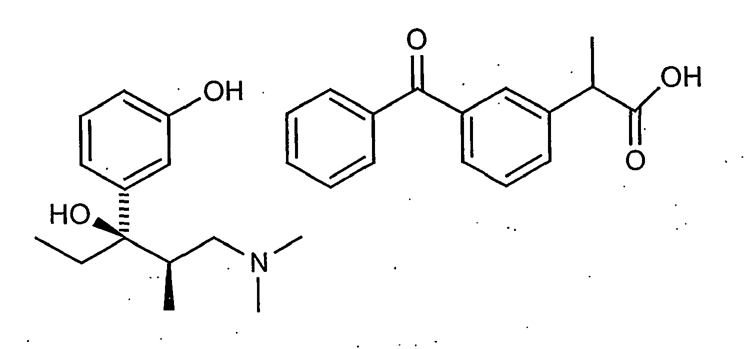

### Beispiel 50

### Salz von M1 mit lsoxicam

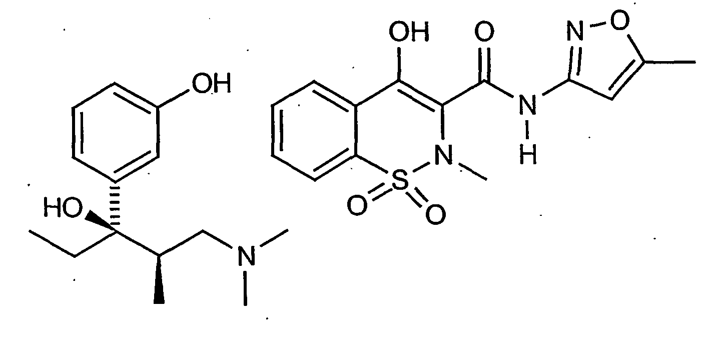

### Beispiel 51

### Salz von M1 mit Piroxicam

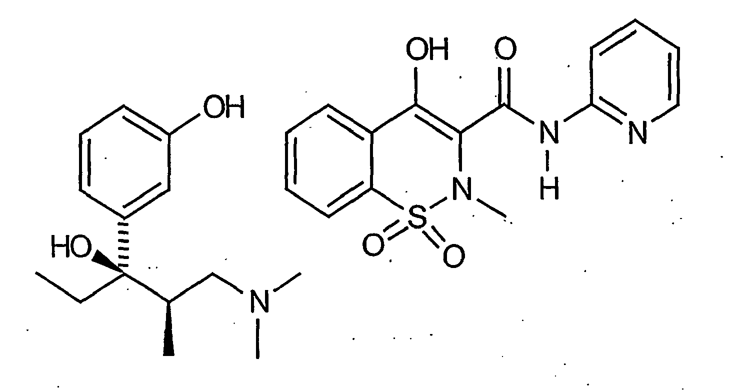

### Beispiel 52

### Salz von M1 mit Phenylbutazon

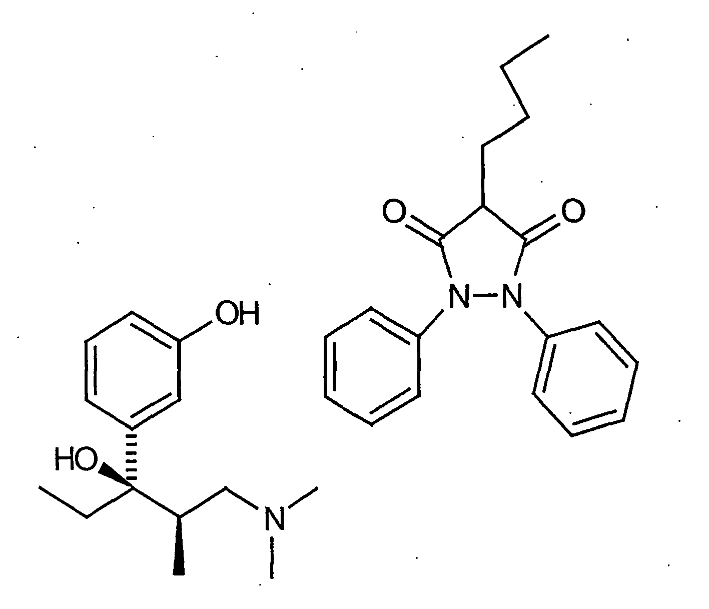

### BEISPIEL 60: ALLGEMEINES VEFAHREN ZUR HERSTELLUNG DER SALZE VON M0

| **Reaktionsgleichung** | |
|---|---|
| 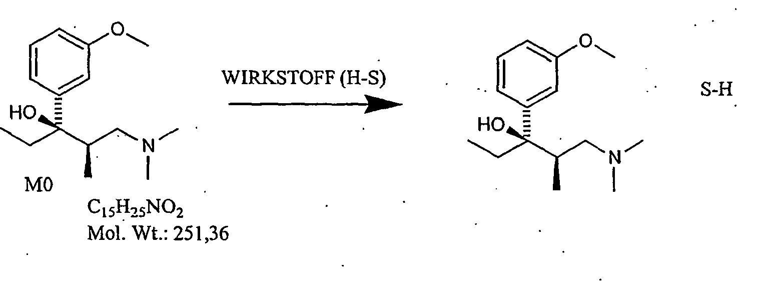 | |

| **Ansatzgröße:** | |
|---|---|
| x mmol M0 | z mg |
| x mmol H-S | y mg |
| | |
| Aceton | |
| **Apparatur:** 50 ml EHK, Rückflusskühler, Magnetrührer | |
| **Durchführung/Reaktionsbedingungen:** Base und Säure einwiegen und in so wenig wie möglich Aceton lösen. Bei RT bis ca. 40°C rühren lassen. Gegebenenfalls Lösungsmittel einengen. | |

### Beispiel 61:

### Salz von M0 und Diclofenac

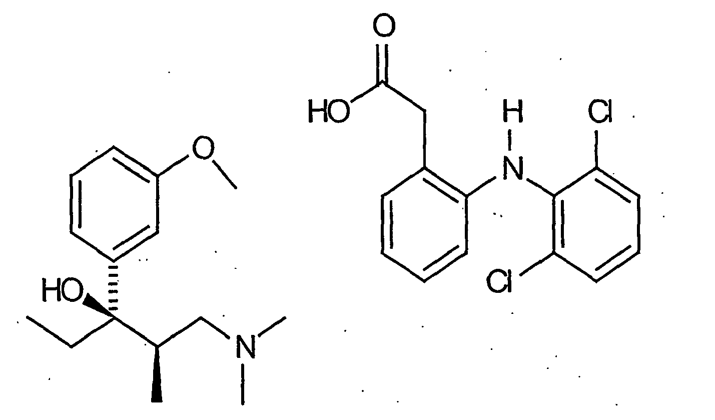

| **Reaktionsgleichung:** | |
|---|---|
| 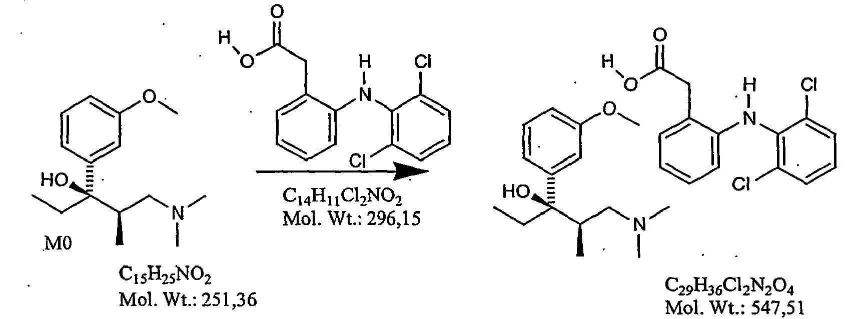 | |

| **Ansatzgröße:** | |
|---|---|
| 1,18 mmol M0 | 297 mg |
| 1,18 mmol Diclofenac | 350 mg |
| | |
| Aceton | |
| **Apparatur:** 50 ml EHK, Magnetrührer, Kühler | |
| **Durchführung/Reaktionsbedingungen:** Base und Säure einwiegen und in Aceton lösen. Bei RT rühren lassen. | |
| **Aufarbeitung:** Nach Einengen erhält man einen weißen Feststoff. | |
| **Auswaage:** 640 mg | 99% d. Theo. |
| **Analytik:** ¹H NMR | Smp:: 56,2 °C |

### Beispiel 62:

### Salz von M0 mit Indomethazin

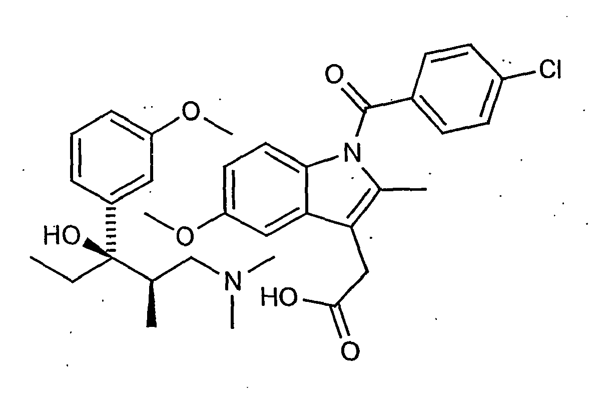

| **Reaktionsgleichung:** | |
|---|---|
| 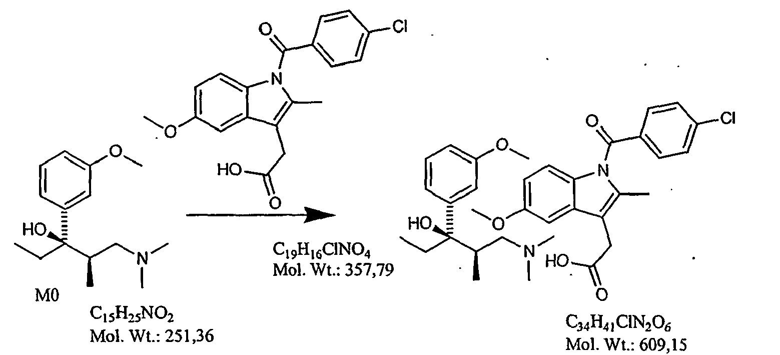 | |

| **Ansatzgröße:** | |
|---|---|
| 1,23 mmol M0 | 297 mg |
| 1,23 mmol Indomethazin | 350 mg |
| | |
| Aceton | |
| **Apparatur:** 50 ml EHK, Magnetrührer, Rückflusskühler | |
| **Durchführung/Reaktionsbedingungen:** Base und Säure einwiegen und in so wenig wie möglich Aceton lösen. 5h bei 35-40°C rühren lassen. Bei RT rühren lassen. | |
| **Aufarbeitung:** | Nach Einengen erhält man einen gelblichen Feststoff |
| **Auswaage:** 740 mg | 99% d. Theo. |
| **Analytik:** ¹H NMR | Smp.:48.3°C |

**Die folgenden Salze der Beispiele 63 bis 72 wurden wie vorhergehend beschrieben (s. Beispiel 60) hergestellt.**

### Beispiel 63

### Salz von M0 mit Ibuprofen

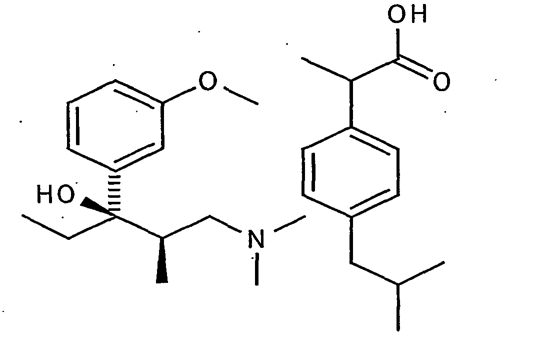

### Beispiel 64

### Salz von M0 mit (s)(+) Ibuprofen

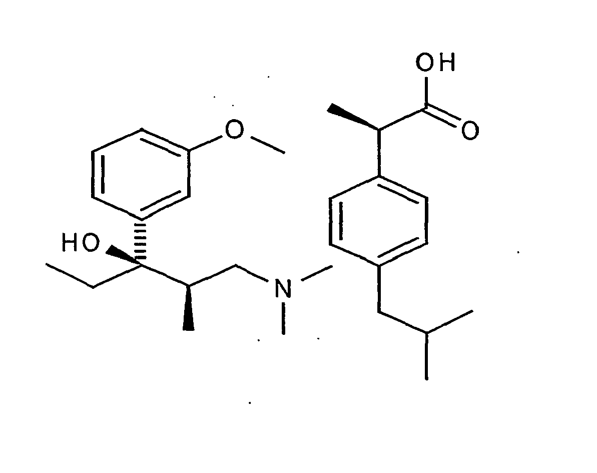

### Beispiel 65

### Salz von M0 mit (S)(+) Naproxen

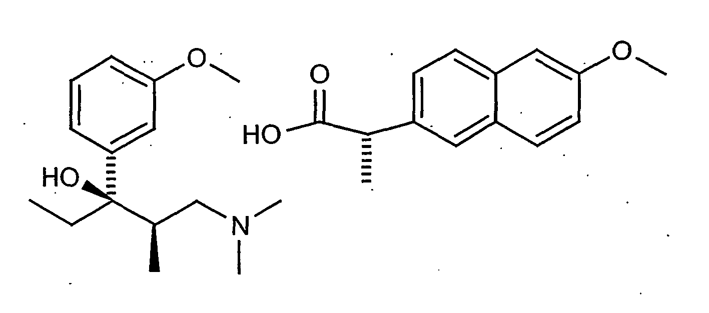

### Beispiel 66

### Salz von M0 mit Acetylsalicylsäure

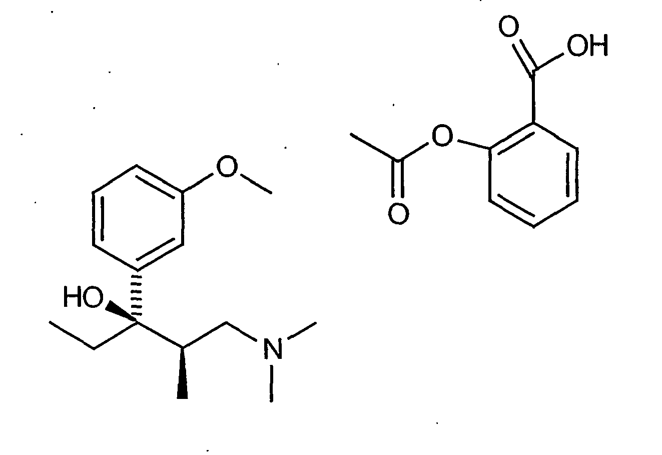

### Beispiel 67

### Salz von M0 mit Dipyron

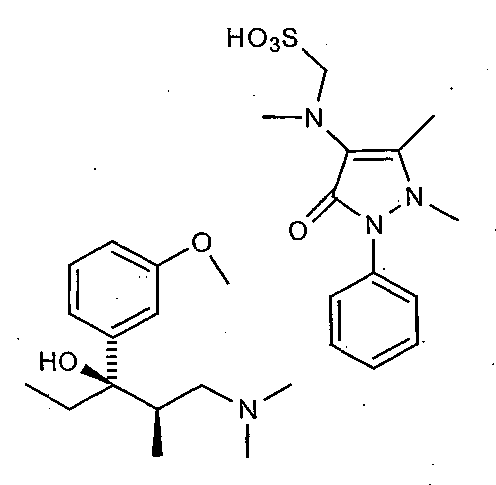

### Beispiel 68

### Salz von M0 mit Ketoprofen

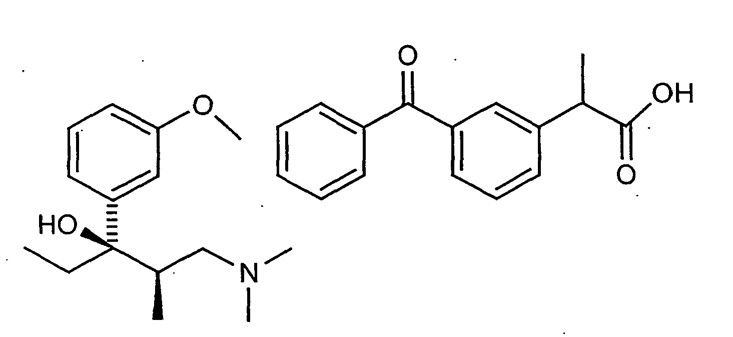

### Beispiel 69

### Salz von M0 mit Isoxicam

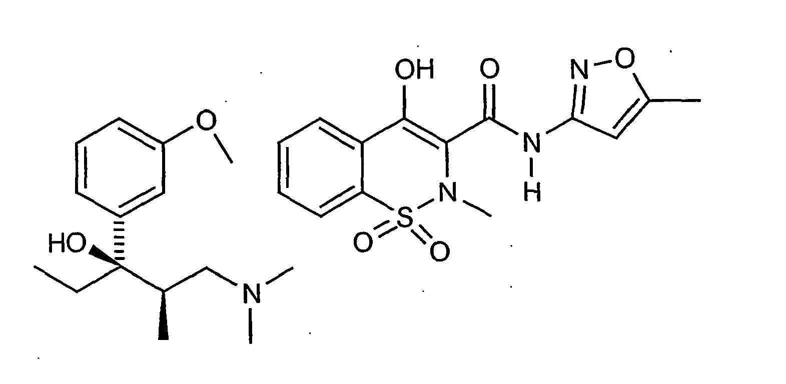

### Beispiel 70

### Salz von M0 mit Flurbiprofen

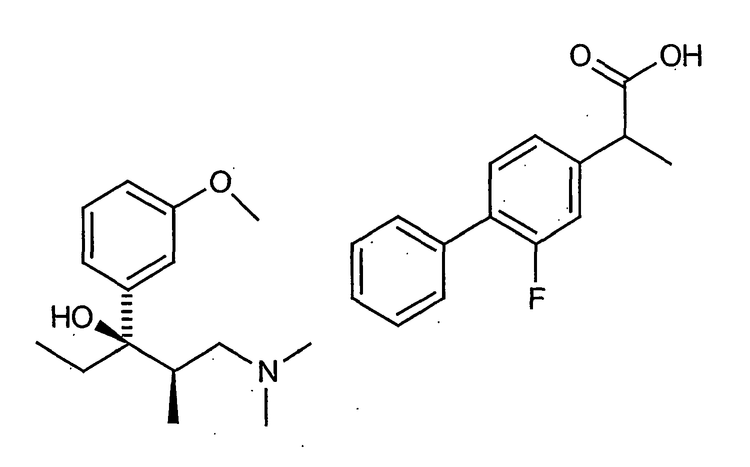

### Beispiel 71

### Salz von M0 mit Piroxicam

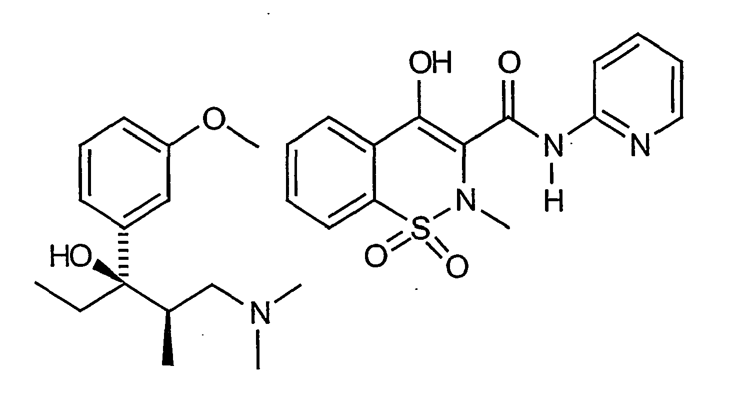

### Beispiel 72

### Salz von M0 mit Phenylbutazon

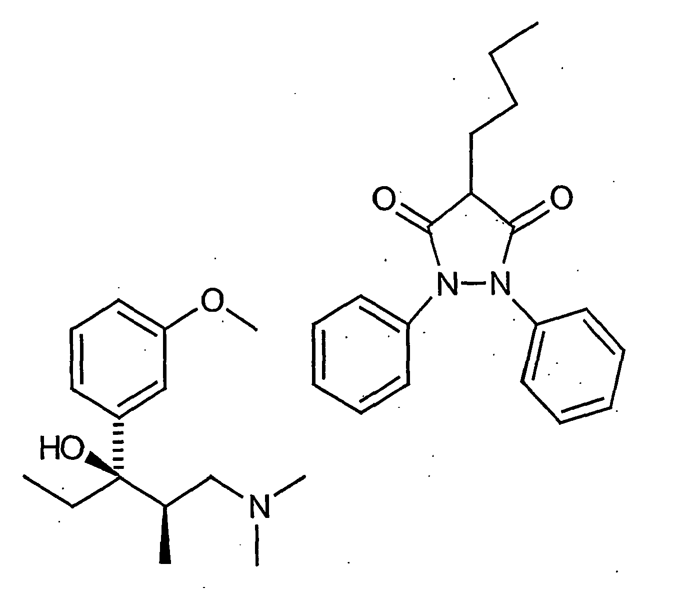

### Beispiel 100: Parenterale Applikationsform

**20 g Wirkstoffsalz** 1-Dimethylamino-3-(3-methoxy-phenyl)-2-methyl-pentan-3-ol mit Diclofenac (Beispiel 61) wird in 1 l Wasser für Injektionszwecke bei Raumtemperatur gelöst und anschließend durch Zugabe von NaCI auf isotone Bedingungen eingestellt.

## Patentansprüche

1. Wirkstoff salz von 1-Dimethylamino-3-(3-methoxy-.phenyl)-2-methyl-pentan-3-ol gebildet aus 1-Dimethylamino-3-(3-methoxy-phenyl)-2-methyl-pentan-3-ol und einem Wirkstoff ausgewählt aus:
• Ibuprofen,
• (S)(+) Ibuprofen,
• (8)(+) Naproxen,
• Diclofenac,
• Acetylsalicylsäure,
• Dipyron,
• Indomethazin,
• Ketoprofen,
• Isoxicam,
• Flurbiprofen,
• Piroxicam oder
• Phenylbutazon,
in Bezug auf 1-Dimethylamino-3-(3-methoxy-phenyl)-2-methyl-pentan-3-ol und/oder den Wirkstoff gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis.

2. Wirkstoffsalz gemäß Anspruch 1 **dadurch gekennzeichnet, dass** es sich um das Wirkstoffsalz von (+)-(2R,3R)-1-Dimethylamino-3-(3-methoxy-phenyl)-2-methyl-pentan-3-ol handelt.

3. Wirkstoffsalz von 3-(3-Dimethylamino-1-ethyl-1-hydroxy-2-methyl-propyl)-phenol gebildet aus 3-(3-Dimethylamino-1-ethyl-1-hydroxy-2-methyl-propyl)-phenol und einem Wirkstoff ausgewählt aus:
• Ibuprofen,
• (S)(+) Ibuprofen,
• (S)(+) Naproxen,
• Diclofenac,
• Acetylsalicylsäure,
• Dipyron,
• Indomethazin,
• Ketoprofen,
• Isoxicam,
• Flurbiprofen,
• Piroxicam oder
• Phenylbutazon,
in Bezug auf 3-(3-Dimethylamino-1-ethyl-1-hydroxy-2-methyl-propyl)-phenol und/oder den Wirkstoff gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis.

4. Wirkstoffsalz gemäß Anspruch 3, **dadurch gekennzeichnet, dass** es sich um das Wirkstoffsalz von (+)-(2R,3R)-3-(3-Dimethylamino-1-ethyl-1-hydroxy-2-methyl-propyl)-phenol handelt.

5. Ester von 3-(3-Dimethylamino-1-ethyl-1-hydroxy-2-methyl-propyl)-phenol gemäß Formel I 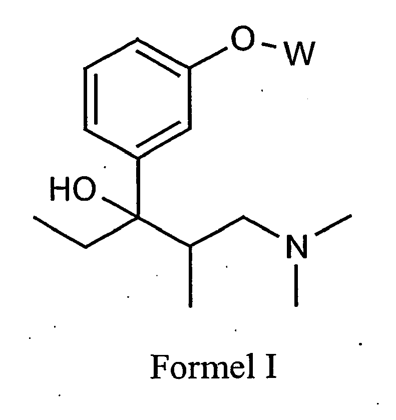 mit W ausgewählt aus dehydroxyliertem
• Ibuprofen,
• (S)(+) Ibuprofen,
• (S)(+) Naproxen,
• Indomethazin,
• Diclofenac,
• Dipyron,
• Flurbiprofen,
• Ketoprofen oder
• Acetylsalicylsäure,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

6. Ester gemäß Anspruch 5, **dadurch gekennzeichnet, daß** die Verbindung in der Konformation der Formel Ib 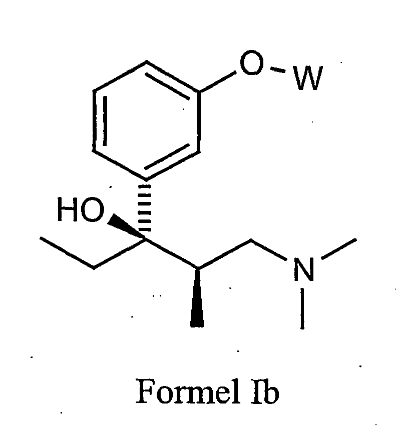 vorliegt.

7. Ester gemäß Formel II 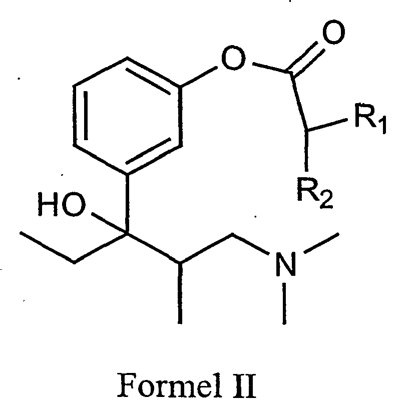 mit R₁ ausgewählt aus Aryl und Heteroaryl, jeweils substituiert oder unsubstituiert,
und R₂ ausgewählt aus H oder C₁₋₃-Alkyl, gesättigt oder ungesättigt, unsubstituiert oder ein- oder mehrfach substituiert,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

8. Ester gemäß Anspruch 7, **dadurch gekennzeichnet, dass** der Ester in der Konformation IIb 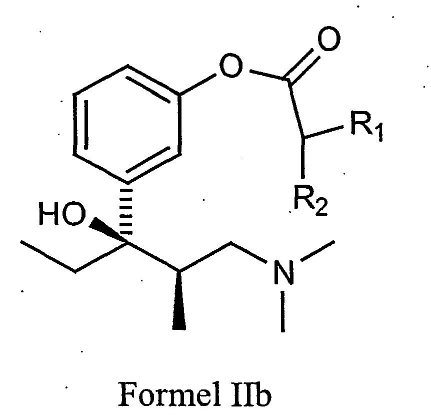 vorliegt.

9. Ester gemäß einem der Ansprüche 7 oder 8, **dadurch gekennzeichnet, dass** R₁ ausgewählt ist aus

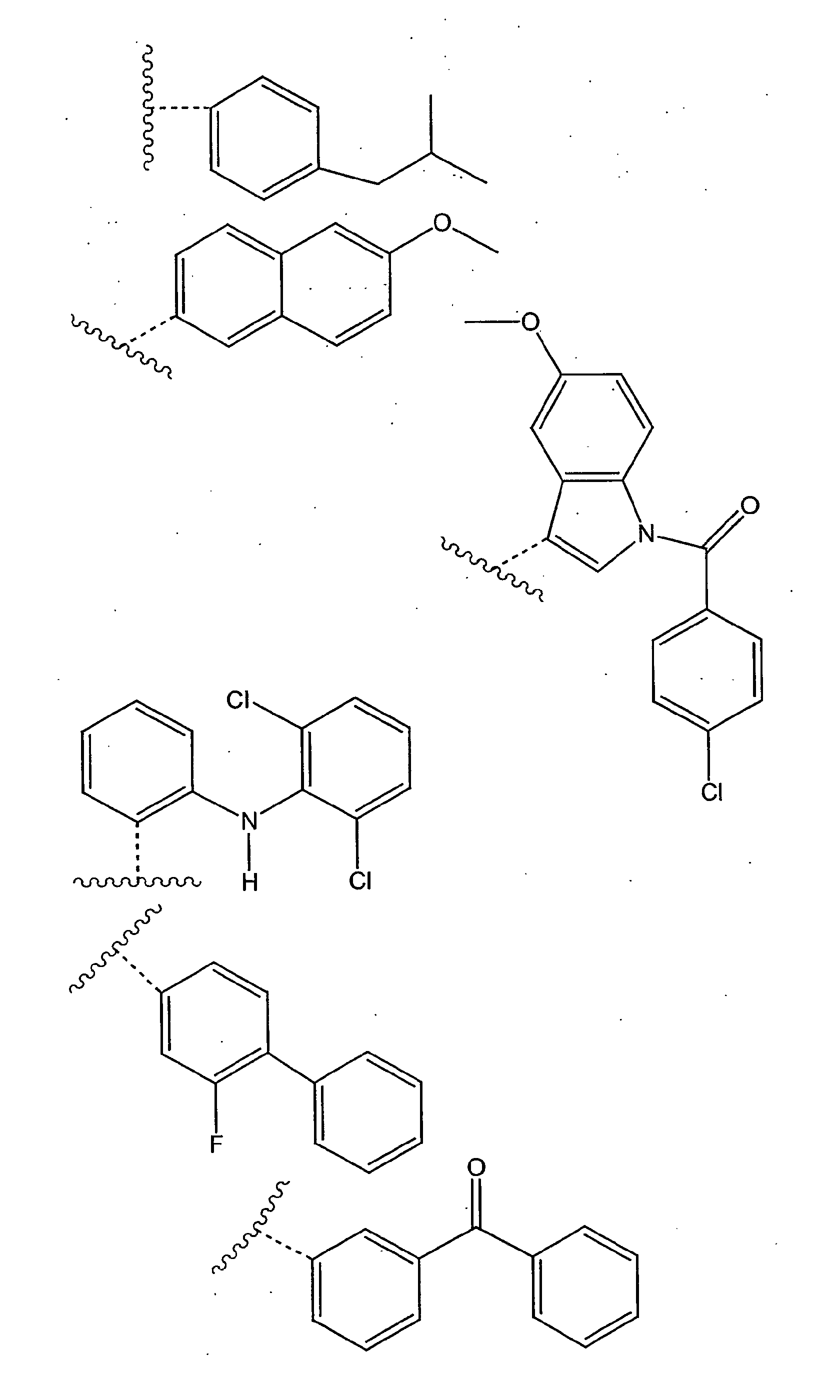

10. Ester gemäß einem der Ansprüche 7 bis 9, **dadurch gekennzeichnet, dass** R₂ ausgewählt ist aus H oder CH₃.

11. Ester gemäß Formel III 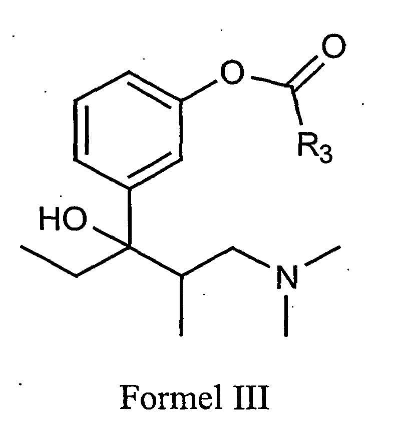 mit R₃ ausgewählt aus Aryl und Heteroaryl, jeweils substituiert oder unsubstituiert,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

12. Ester gemäß Anspruch 11, **dadurch gekennzeichnet, dass** der Ester in der Konformation IIIb 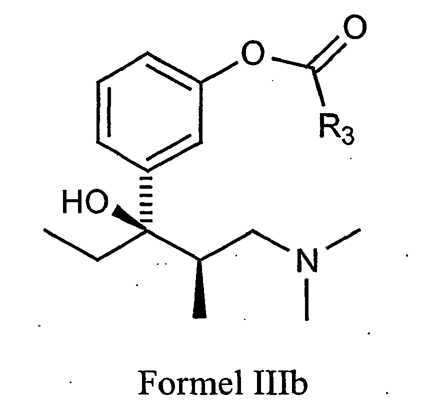 vorliegt.

13. Ester gemäß einem der Ansprüche 11 oder 12, **dadurch gekennzeichnet, dass** R₃ ausgewählt ist aus

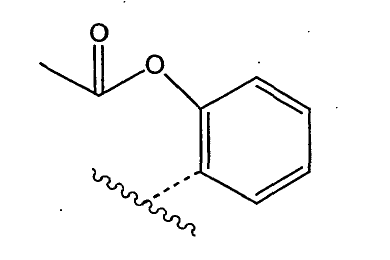

14. Ester gemäß Formel IV 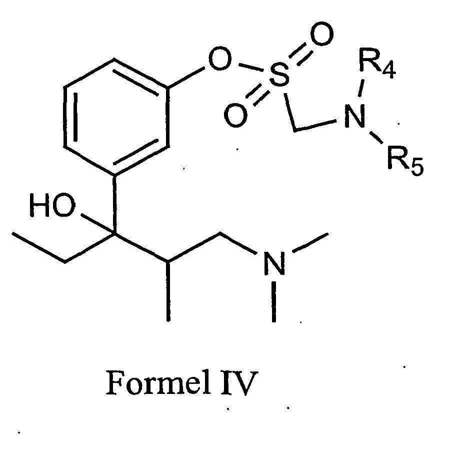 mit R₄ ausgewählt aus Aryl und Heteroaryl, jeweils substituiert oder unsubstituiert,
und R₅- ausgewählt aus H oder C₁₋₃-Alkyl, gesättigt oder ungesättigt, unsubstituiert oder ein- oder mehrfach substituiert,
gegebenenfalls in Form der Racemate, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

15. Ester gemäß Anspruch 14, **dadurch gekennzeichnet, dass** der Ester in der Konformation IVb 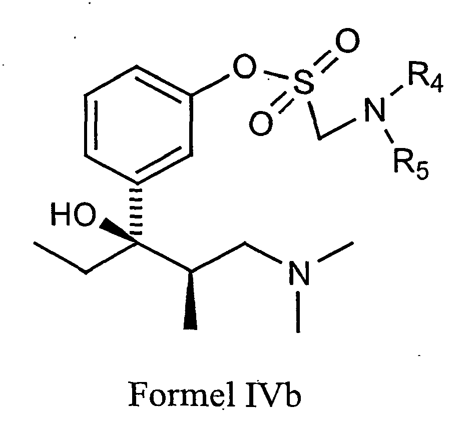 vorliegt.

16. Ester gemäß einem der Ansprüche 14 oder 15, **dadurch gekennzeichnet, dass** R₄ ausgewählt ist aus

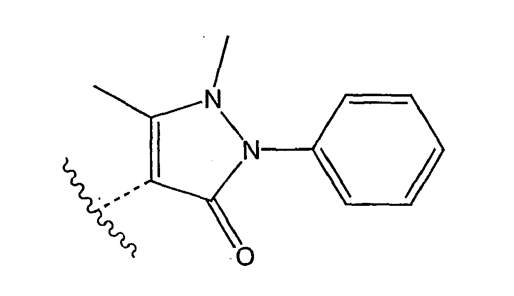

17. Ester gemäß einem der Ansprüche 14 bis 16, **dadurch gekennzeichnet, dass** R₅ ausgewählt ist aus H oder CH₃.

18. Arzneimittel enthaltend mindestens ein Wirkstoffsalz gemäß einem der Ansprüche 1 bis 4 oder einen Ester gemäß einem der Ansprüche 5 bis 17 sowie gegebenenfalls geeignete Zusatz- und/oder HilfsstofFe.

19. Verwendung eines Wirkstoffsalzes gemäß einem der Ansprüche 1 bis 4 öder eines Esters gemäß einem der Ansprüche 5 bis 17 zur Herstellung eines Arzneimittels zur Behandlung von vermehrtem Harndrang bzw. Harninkontinenz.

20. Verwendung eines Wirkstoffsalzes gemäß einem der Ansprüche 1 bis 4 oder eines Esters gemäß einem der Ansprüche 5 bis 17 zur Herstellung eines Arzneimittels zur Behandlung von Schmerz, insbesondere von chronischem, viszeralem, neuropathischem oder akutem Schmerz oder Entzündungsschmerz.

## Claims

1. Active constituent salt of 1-dimethylamino-3-(3-methoxyphenyl)-2-methylpentan-3-ol formed from 1-dimethylamino-3-(3-methoxyphenyl)-2-methylpentan-3-ol and an active constituent selected from:
• Ibuprofen,
• (s) (+) Ibuprofen,
• (S) (+) Naproxen,
• Diclofenac,
• acetylsalicylic acid,
• Dipyron,
• Indomethacin,
• Ketoprofen
• Isoxicam,
• Flurbiprofen,
• Piroxicam or
• phenylbutazone,
with respect to 1-dimethylamino-3-(3-methoxyphenyl)-2-methylpentan-3-ol and/or the active constituent optionally in the form of the racemates, pure stereoisomers, in particular enantiomers or diastereomers, or in the form of mixtures of the stereoisomers, in particular the enantiomers or diastereomers, in an arbitrary mixture ratio.

2. Active constituent salt according to claim 1, **characterised in that** it is the active constituent salt of (+)-(2R,3R)-1-dimethylamino-3-(3-methoxyphenyl)-2-methylpentan-3-ol.

3. Active constituent salt of 3-(3-dimethylamino-l-ethyl-1-hydroxy-2-methylpropyl)-phenol formed from 3-(3-dimethylamino-1-ethyl-1-hydroxy-2-methylpropyl)-phenol and an active constituent selected from:
• Ibuprofen,
• (s) (+) Ibuprofen,
• (S)(+)Naproxen,
• Diclofenac,
• acetylsalicylic acid,
• Dipyron,
• Indomethacin,
• Ketoprofen
• Isoxicam,
• Flurbiprofen,
• Piroxicam or
• phenylbutazone,
with respect to 3-(3-dimethylamino-1-ethyl-1-hydroxy-2-methylpropyl)-phenol and/or the active constituent optionally in the form of the racemates, pure stereoisomers, in particular enantiomers or diastereomers, or in the form of mixtures of the stereoisomers, in particular the enantiomers or diastereomers, in an arbitrary mixture ratio.

4. Active constituent salt according to claim 3, **characterised in that** it is the active constituent salt of (+)-(2R,3R)-3-(3-dimethylamino-1-ethyl-1-hydroxy-2-methylpropyl)-phenol.

5. Ester of 3-(3-dimethylamino-1-ethyl-1-hydroxy-2-methylpropyl)-phenol according to formula I: 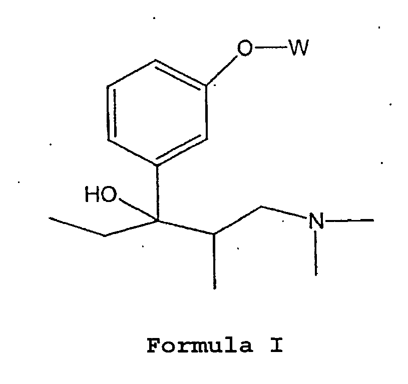 where W is selected from dehydroxylated:
• Ibuprofen,
• (s) (+) Ibuprofen,
• (S) (+)Naproxen,
• Indomethacin,
• Diclofenac,
• Dipyron,
• Flurbiprofen,
• Ketoprofen or
• acetylsalicylic acid,
optionally in the form of the racemates, pure stereoisomers, in particular enantiomers or diastereomers, or in the form of mixtures of the stereoisomers, in particular enantiomers or diastereomers, in an arbitrary mixture ratio; in the prepared form or in the form of their acids or their bases or in the form of their salts, in particular physiologically compatible salts, or in the form of their solvates, in particular hydrates.

6. Ester according to claim 5, **characterised in that** the compound is present in the conformation of the formula Ib

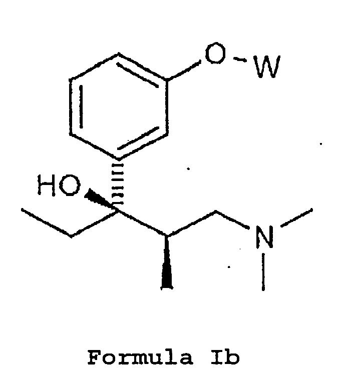

7. Ester according to formula II 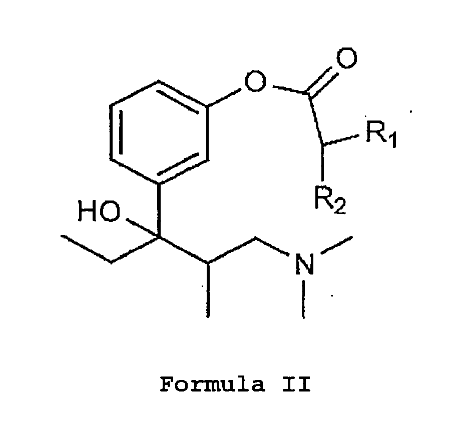 where R₁ is selected from aryl and heteroaryl, in each case substituted or unsubstituted,
and R₂ is selected from H or C₁₋₃-alkyl, which is saturated or unsaturated, unsubstituted or singly or multiply substituted,
optionally in the form of the racemates, pure stereoisomers, in particular enantiomers and diastereomers, or in the form of mixtures of the stereoisomers, in particular of the enantiomers or diastereomers, in an arbitrary mixture ratio; in the prepared form or in the form of their acids or their bases or in the form of their salts, in particular physiologically compatible salts, or in the form or their solvates, in particular the hydrates.

8. Ester according to claim 7, **characterised in that** the ester is present in the conformation lib

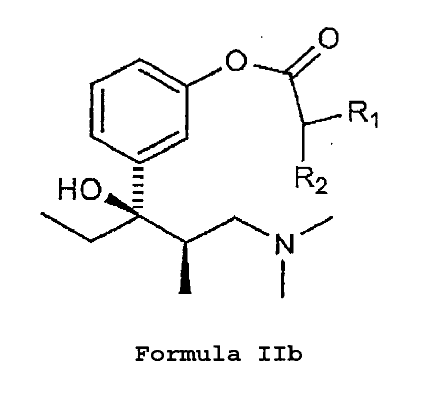

9. Ester according to one of claims 7 and 8, **characterised in that** R₁ is selected from

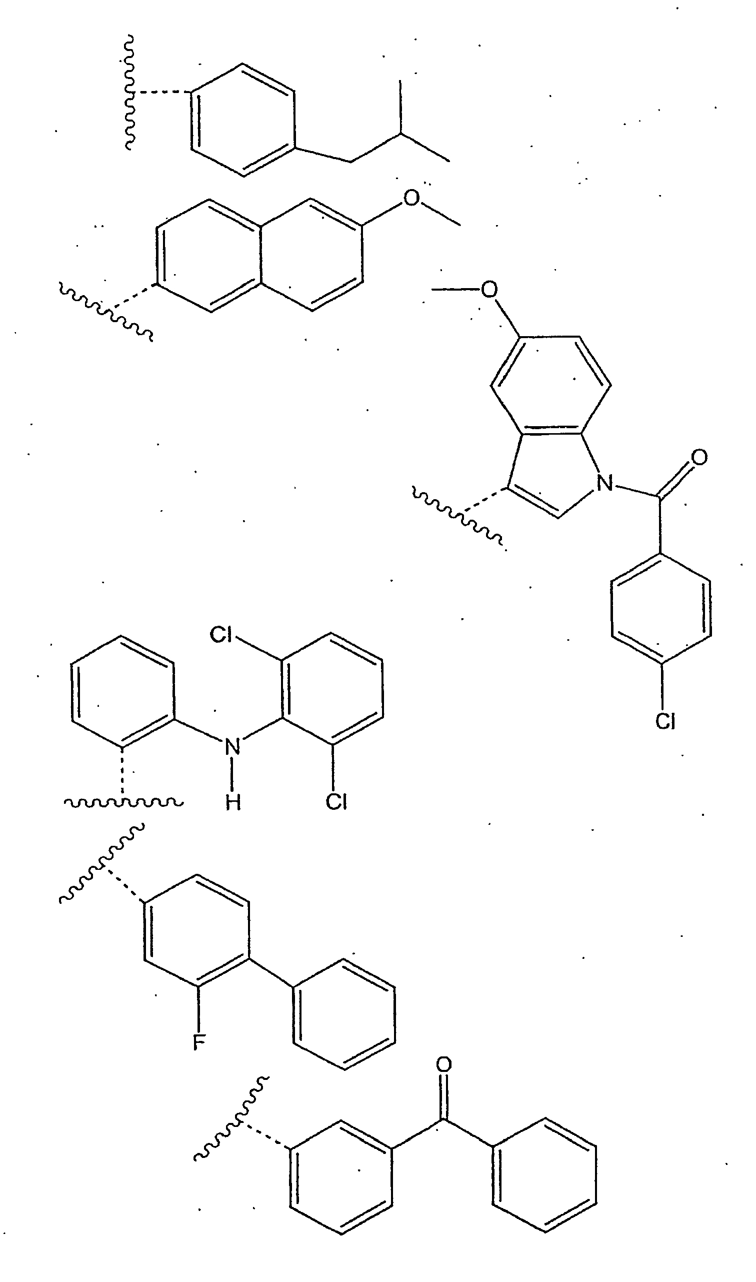

10. Ester according to one of claims 7 to 9, **characterised in that** R₂ is selected from H or CH₃.

11. Ester according to formula III 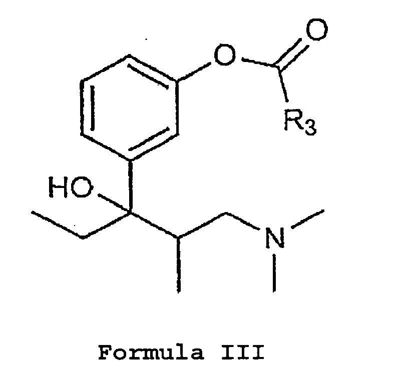 where R₃ is selected from aryl and heteroaryl, in each case substituted or unsubstituted,
optionally in the form of the racemates, pure stereoisomers, in particular enantiomers or diastereomers, or in the form of mixtures of the stereoisomers, in particular enantiomers or diastereomers, in an arbitrary mixture ratio; in the prepared form or in the form of their acids or their bases or in the form of their salts, in particular physiologically compatible salts, or in the form of their solvates, in particular hydrates.

12. Ester according to claim 11, **characterised in that** the ester is present in the conformation IIIb

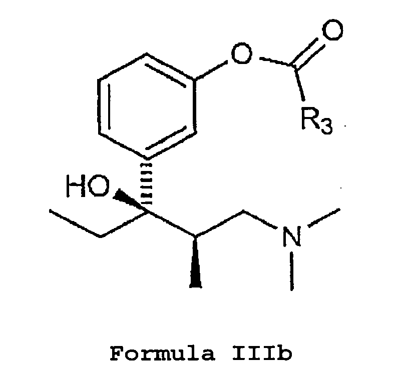

13. Ester according to one of claims 11 and 12, **characterised in that** R₃ is selected from

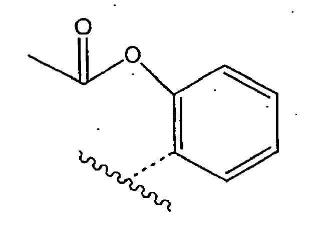

14. Ester according to formula IV 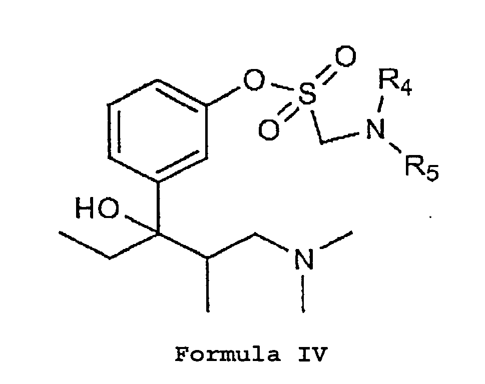 where R₄ is selected from aryl and heteroaryl, in each case substituted or unsubstituted,
and R₅ is selected from H or C₁₋₃-alkyl, which is saturated or unsaturated, unsubstituted or singly or multiply substituted,
optionally in the form of the racemates, pure stereoisomers, in particular enantiomers or diastereomers, or in the form of mixtures of the stereoisomers, in particular enantiomers and diastereomers, in an arbitrary mixture ratio; in the prepared form or in the form of their acids or their bases or in the form of their salts, in particular physiologically compatible salts, or in the form of their solvates, in particular hydrates.

15. Ester according to claim 14, **characterised in that** the ester is present in the conformation IVb

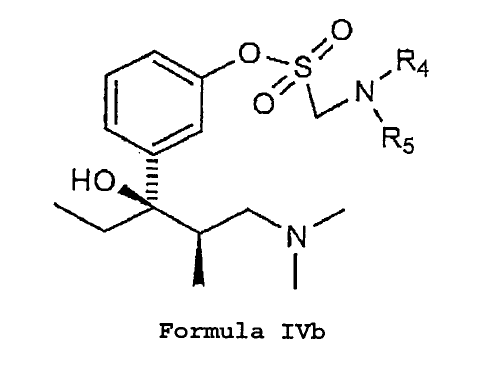

16. Ester according to one of claims 14 and 15, **characterised in that** R₄ is selected from

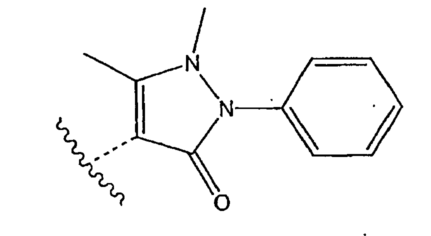

17. Ester according to one of claims 14 to 16, **characterised in that** R₅ is selected from H or CH₃.

18. Medicament containing at least one active constituent salt according to one of claims 1 to 4 or an ester according to one of claims 5 to 17, as well as optionally suitable additives and/or auxiliary substances.

19. Use of an active constituent salt according to one of claims 1 to 4 or an ester according to one of claims 5 to 17 for the production of a medicament for treating increased urinary urgency or urinary incontinence.

20. Use of an active constituent salt according to one of claims 1 to 4 or of an ester according to one of claims 5 to 17 for the production of a medicament for treating pain, in particular chronic, visceral, neuropathic or acute pain or inflammation pain.

## Revendications

1. Sel de substance active du 1-diméthylamino-3-(3-méthoxyphényl)-2-méthylpentane-3-ol formé à partir de 1-diméthylamino-3-(3-méthoxyphényl)-2-méthylpentane-3-ol et d'une substance active choisie entre :
• ibuprofène
• (S)(+)-ibuprofène
• (S)(+)-naproxène
• diclofénac
• acide acétylsalicylique
• dipyrone
• indométhazine
• kétoprofène
• isoxicam
• flurbiprofène
• piroxicam ou
• phénylbutazone
en ce qui concerne le 1-diméthylamino-3-(3-méthoxy-phényl)-2-méthyl-pentane-3-ol et/ou la substance active, le cas échéant sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque.

2. Sel de substance active suivant la revendication 1, **caractérisé en ce qu'**il s'agit du sel de substance active du (+)-(2R,3R)-1-diméthylamino-3-(3-méthoxyphényl)-2-méthyl-pentane-3-ol.

3. Sel de substance active de 3-(3-diméthylamino-1-éthyl-1-hydroxy-2-méthyl-propyl)-phénol, formé de 3-(3-diméthylamino-1-éthyl-1-hydroxy-2-méthyl-propyl)-phénol et d'une substance active choisie entre :
• ibuprofène
• (S) (+) -ibuprofène
• (S)(+)-naproxène
• diclofénac
• acide acétylsalicylique
• dipyrone
• indométhazine
• kétoprofène
• isoxicam
• flurbiprofène
• piroxicam ou
• phénylbutazone
en ce qui concerne le 3-(3-diméthylamino-1-éthyl-1-hydroxy-2-méthyl-propyl)-phénol et/ou la substance active, le cas échéant sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque.

4. Sel de substance active suivant la revendication 3, **caractérisé en ce qu'**il s'agit du sel de substance active du (+)-(2R,3R)-3-(3-diméthylamino-1-éthyl-1-hydroxy-2-méthyl-propyl)-phénol.

5. Ester de 3-(3-diméthylamino-1-éthyl-1-hydroxy-2-méthyl-propyl)-phénol répondant à la formule I 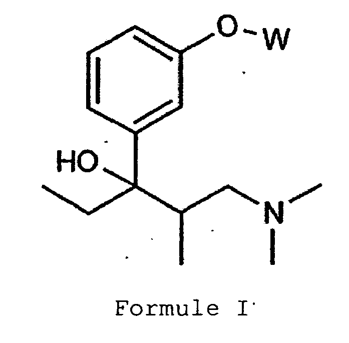 dans laquelle W est choisi entre
• ibuprofène
• (S) (+) -ibuprofène
• (S) (+)-naproxène
• indométhazine
• diclofénac
• dipyrone
• flurbiprofène
• kétoprofène ou
• acide acétylsalicylique,
chacun déshydroxylé, le cas échéant sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ; sous la forme représentée ou sous forme de leurs acides ou de leurs bases ou sous forme de leurs sels, en particulier des sels physiologiquement acceptables, ou sous forme de leurs produits de solvatation, en particulier des hydrates.

6. Ester suivant la revendication 5, **caractérisé en ce que** le composé se présente avec la conformation de la formule Ib

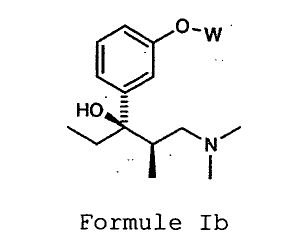

7. Ester répondant à la formule II 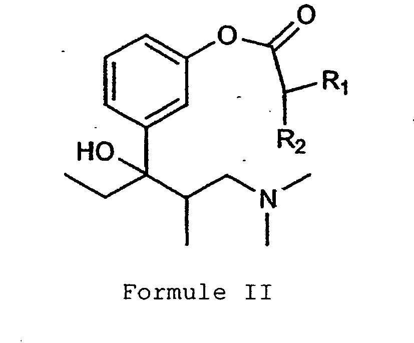 dans laquelle R₁ est choisi entre un reste aryle et un reste hétéroaryle, chacun substitué ou non substitué,
et R₂ est choisi entre H ou un reste alkyle en C₁ à C₃ saturé ou non saturé, non substitué ou substitué une ou plusieurs fois,
éventuellement sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
sous la forme représentée ou sous forme de leurs acides ou de leurs bases ou sous forme de leurs sels, en particulier des sels physiologiquement acceptables, ou sous forme de leurs produits de solvatation, en particulier des hydrates.

8. Ester selon la revendication 7, **caractérisé en ce qu'**il se présente avec la conformation IIb

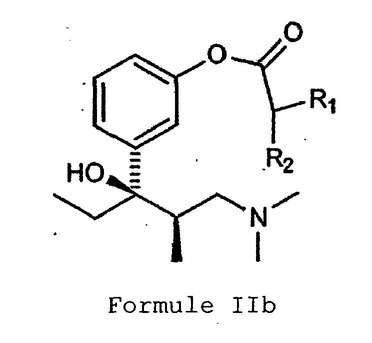

9. Ester selon l'une des revendications 7 ou 8, **caractérisé en ce que** R₁ est choisi entre

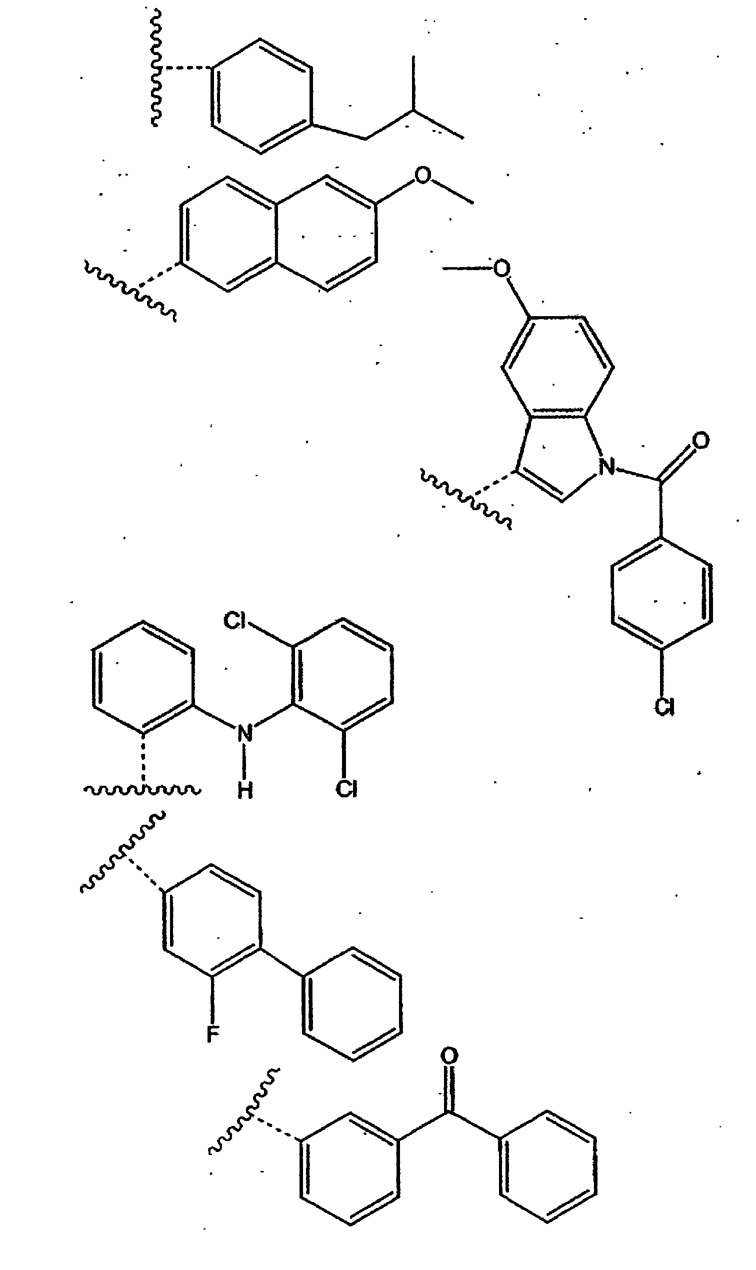

10. Ester selon l'une des revendications 7 à 9, **caractérisé en ce que** R₂ est choisi entre H ou CH₃.

11. Ester répondant à la formule III 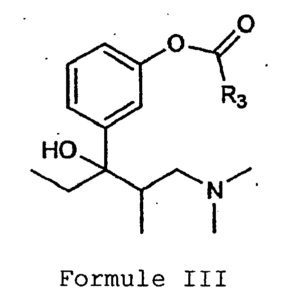 dans laquelle R₃ est choisi entre un reste aryle et un reste hétéroaryle, chacun substitué ou non substitué, éventuellement sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
sous la forme représentée ou sous forme de leurs acides ou de leurs bases ou sous forme de leurs sels, en particulier des sels physiologiquement acceptables, ou sous forme de leurs produits de solvatation, en particulier des hydrates.

12. Ester selon la revendication 11, **caractérisé en ce qu'**il se présente avec la conformation IIIb

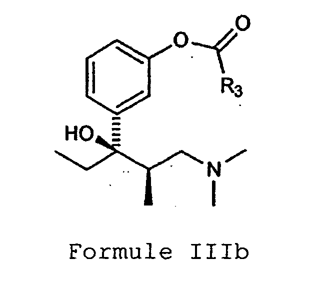

13. Ester selon l'une des revendications 11 ou 12, **caractérisé en ce que** R₃ est choisi comme groupe

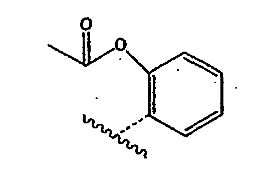

14. Ester répondant à la formule IV 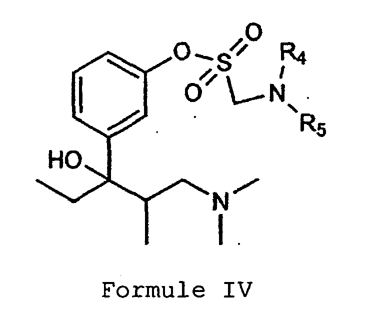 dans laquelle R₄ est choisi entre un reste aryle et un reste hétéroaryle, chacun substitué ou non substitué,
et R₅ est choisi entre H ou un groupe alkyle en C₁ à C₃, saturé ou non saturé, non substitué ou substitué une ou plusieurs fois,
le cas échéant sous forme des racémates, des stéréoisomères purs, en particulier des énantiomères ou des diastéréoisomères, ou sous forme de mélanges des stéréoisomères, en particulier des énantiomères ou des diastéréoisomères, dans un rapport de mélange quelconque ;
sous la forme représentée ou sous forme de leurs acides ou de leurs bases ou sous forme de leurs sels, en particulier des sels physiologiquement acceptables, ou sous forme de leurs produits de solvatation, en particulier des hydrates.

15. Ester selon la revendication 14, **caractérisé en ce qu'**il se présente avec la conformation IVb

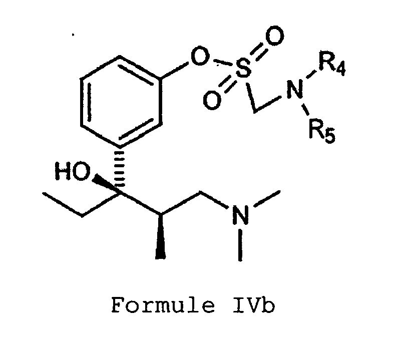

16. Ester selon l'une des revendications 14 ou 15, **caractérisé en ce que** R₄ est choisi comme groupe

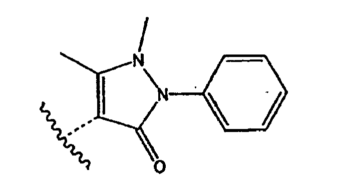

17. Ester selon l'une des revendications 14 à 16, **caractérisé en ce que** R₅ est choisi entre H ou CH₃.

18. Médicament contenant au moins un sel de substance active selon l'une des revendications 1 à 4 ou un ester selon l'une des revendications 5 à 17 ainsi que, le cas échéant, des additifs et/ou des substances auxiliaires convenables.

19. Utilisation d'un sel de substance active selon l'une des revendications 1 à 4 ou d'un ester selon l'une des revendications 5 à 17 pour la préparation d'un médicament destiné au traitement de la répétition du besoin impérieux d'uriner ou de l'incontinence d'urine.

20. Utilisation d'un sel de substance active selon l'une des revendications 1 à 4 ou d'un ester selon l'une des revendications 5 à 17 pour la préparation d'un médicament destiné au traitement de la douleur, en particulier d'une douleur chronique, viscérale, neuropathique ou aiguë ou d'une douleur inflammatoire.
